# EUROPEAN PATENT APPLICATION

(11) **EP 4 844 378 A1**
(43) Date of publication of application: **25.11.2026**
(21) Application number: 25741744.4
(22) Date of filing: 08.01.2025
(51) Int. Cl.: G01N 21/359, C12M 1/34, C12Q 1/06, G01N 15/01

(54) **OBSERVATION DEVICE AND OBSERVATION METHOD**

(30) Priority: 17.01.2024 JP 2024005456
(71) Applicant: Sumitomo Electric Industries, Ltd., Osaka-shi, Osaka 541-0041 (JP); NATIONAL UNIVERSITY CORPORATION TOKAI NATIONAL HIGHER EDUCATION AND RESEARCH SYSTEM, Nagoya-shi, Aichi 464-8601 (JP)
(72) Inventor: SUGIYAMA Yoko, Osaka-shi, Osaka 541-0041 (JP); SUGANUMA Hiroshi, Osaka-shi, Osaka 541-0041 (JP); SANO Tomomi, Osaka-shi, Osaka 541-0041 (JP); KATO Ryuji, Nagoya-shi, Aichi 464-8601 (JP); TANAKA Kenjiro, Nagoya-shi, Aichi 464-8601 (JP)
(74) Representative: Boult Wade Tennant LLP
(86) International application number: PCT/JP2025/000379
(87) International publication number: WO 2025/154611

(57) **Abstract**

An observation device according to the present disclosure is for observing a cell suspension including a plurality of cells. The observation device includes: at least one irradiation unit that is disposed at a position facing the cell suspension and irradiates the cell suspension with irradiation light that has at least one wavelength included in the ranges of 650 nm to 850 nm and 1060 nm to 1090 nm; at least one light receiving unit that is disposed at a position facing the cell suspension and receives emission light emitted from the cell suspension as a result of irradiating the cell suspension with the irradiation light; and an analysis unit that is communicatively connected to the light receiving unit and determines a parameter relating to a cell concentration of the cells in the cell suspension on the basis of an intensity of the emission light.

## Description

### Technical Field

The present disclosure relates to an observation device and an observation method. Priority is claimed on Japanese Patent Application No. 2024-005456, filed January 17, 2024, the content of which is incorporated herein by reference.

### Background Art

For example, in the field of regenerative medicine, research has been conducted into disease treatments using cells or cell clusters, and into drug discovery evaluation using cells as an alternative to laboratory animals. Cells used in these fields are produced by culturing and growing human or animal-derived cells. In the process of culturing cells, cells are housed in a culture vessel together with a culture medium. The environment within this culture vessel is controlled and managed to grow the cells. Culturing ends when a parameter such as a cell concentration or a cell number of a cell suspension in the vessel reaches a target value.

As methods for determining whether the cell concentration or cell number has reached the target value, for example, a method for collecting and injecting a cell suspension into a counting chamber and counting a cell number through visual observation using a microscope, and a method for collecting and injecting a cell suspension into a cell counter and mechanically counting a cell number through image processing of the cell counter are known. As another method, a method for estimating a cell concentration using, for example, a turbidimeter is also conceivable (see, for example, Patent Literature 1.) In this method, a cell concentration is estimated by measuring an intensity of emission light produced by irradiating a cell suspension with light, and using the fact that the measured light intensity changes depending on the cell concentration.

### Citation List

### Patent Literature

[Patent Literature 1] Japanese Unexamined Patent Publication No. 2023-144814

### Summary of Invention

An observation device of the present disclosure is an observation device for observing a cell suspension containing a plurality of cells. The observation device includes: at least one irradiation unit that is disposed at a position facing the cell suspension and irradiates the cell suspension with irradiation light having at least one wavelength included in the ranges of 650 nm to 850 nm and 1060 nm to 1090 nm; at least one light receiving unit that is disposed at a position facing the cell suspension and receives emission light emitted from the cell suspension as a result of irradiating the cell suspension with the irradiation light; and an analysis unit that is communicatively connected to the light receiving unit and determines a parameter relating to a cell concentration of cells in the cell suspension on the basis of an intensity of the emission light.

### Brief Description of Drawings

Fig. 1 is a diagram showing a configuration of an observation device according to a first embodiment.
Fig. 2 is a diagram showing a hardware configuration of an analysis unit in Fig. 1.
Fig. 3 is a diagram showing a functional configuration of the analysis unit in Fig. 1.
Fig. 4 is a graph showing a calibration curve held by the analysis unit in Fig. 1.
Fig. 5 is a graph showing a spectrum of an amount of change in light intensity of cells.
Fig. 6 is a graph showing a spectrum of an amount of change in light intensity of a culture medium.
Fig. 7 is a flowchart showing an example of an observation method performed using the observation device of Fig. 1.
Fig. 8 is a diagram showing a modified example of the observation device of Fig. 1.
Fig. 9 is a graph showing a calibration curve held by an analysis unit in Fig. 8.
Fig. 10 is a diagram showing a modified example of the observation device of Fig. 1.
Fig. 11 is a diagram showing a configuration of an observation device of a second embodiment.
Fig. 12 is a diagram showing a functional configuration of an analysis unit in Fig. 11.
Fig. 13 is a graph showing a calibration curve held by the analysis unit in Fig. 11.
Fig. 14 is a graph for describing how to obtain a Rayleigh scattering coefficient in the analysis unit in Fig. 11.
Fig. 15 is a graph for describing a wavelength range suitable for observing a state of a cell suspension.
Fig. 16 is a diagram showing a modified example of the observation device of Fig. 11.
Fig. 17 is a graph for describing how to obtain a Rayleigh scattering coefficient in an analysis unit in Fig. 16.
Fig. 18 is a diagram showing a modified example of the observation device of Fig. 11.
Fig. 19 is a diagram showing a modified example of the observation device of Fig. 11.
Fig. 20 is a diagram illustrating a modified example of the observation device of Fig. 11.

### Description of Embodiments

### Problems to be Solved by Present Disclosure

In the method using a counting chamber or a cell counter described above, the number of cells cannot be counted while the cells are suspended three-dimensionally in the cell suspension, and thus tasks of collecting and injecting the cell suspension into a container that encloses it in a flat space or into a measurement unit in the device are required. This method makes it difficult to count the number of cells efficiently. In the method using a turbidimeter, the cell suspension is irradiated with light, and the number of cells is estimated on the basis of the intensity of the emission light from the cell suspension. A mixture of light absorption by the cells and the culture medium and light scattering by the cells is reflected in the intensity of the emission light. For this reason, the intensity of absorption or scattering can be observed by observing an amount of change or a relative value of the emission light intensity relative to the irradiation light intensity (hereinafter, an "amount of change or relative value of light intensity relative to the irradiation light intensity" will be described as an "amount of change in light intensity"). In the following, the "intensity of absorption" and the "absorption intensity" indicate an "amount of change in light intensity due to absorption," and the "intensity of scattering" and "scattering intensity" indicate an "amount of change in light intensity due to scattering." Among these phenomena, an amount of change in emission light intensity due to scattering depends on a cell concentration of a cell suspension and can be used to estimate the cell concentration. However, from the obtained amount of change in light intensity, it is difficult to distinguish between the amount of change in light intensity due to scattering and the amount of change in light intensity due to absorption, and thus it is difficult to accurately obtain the cell concentration of the cell suspension using light intensity.

The present disclosure provides an observation device and an observation method that can accurately and efficiently observe a state of a cell suspension.

### Effects of Present Disclosure

According to the present disclosure, an observation device and an observation method that can accurately and efficiently observe a state of a cell suspension are provided.

### Description of Embodiments of Present Disclosure

First, aspects of the present disclosure will be listed and described.
(1) An observation device of the present disclosure is an observation device for observing a cell suspension containing a plurality of cells. The observation device includes: at least one irradiation unit that is disposed at a position facing the cell suspension and irradiates the cell suspension with irradiation light having at least one wavelength included in the ranges of 650 nm to 850 nm and 1060 nm to 1090 nm; at least one light receiving unit that is disposed at a position facing the cell suspension and receives emission light emitted from the cell suspension as a result of irradiating the cell suspension with the irradiation light; and an analysis unit that is communicatively connected to the light receiving unit and obtains a parameter relating to a cell concentration of cells in the cell suspension on the basis of an intensity of the emission light.
   In general, when light is radiated onto a cell suspension, phenomena such as light scattering and absorption can occur in the cell suspension. A mixture of these phenomena is reflected in an intensity of light emitted from the cell suspension in response to light irradiation. For this reason, the intensity of absorption and scattering can be observed by observing an amount of change in light intensity. An amount of change in light intensity due to the scattering among these phenomena depends on parameters relating to a cell concentration in the cell suspension. An amount of change in light intensity due to the absorption depends on chemical reactions in the cell suspension. Accordingly, in order to accurately obtain the parameters relating to the cell concentration, the amount of change in light intensity due solely to the scattering may be acquired. After extensive investigation into methods for acquiring an amount of change in light intensity due solely to scattering, the inventors have found that it is effective to irradiate a cell suspension with irradiation light having at least one wavelength included in the ranges of 650 nm to 850 nm and 1060 nm to 1090 nm. In the wavelength ranges of 650 nm to 850 nm and 1060 nm to 1090 nm, light absorption by a culture medium in which cells are disposed is sufficiently small as compared to light scattering by the cells, and thus the effect of light absorption by the culture medium on the intensity of the emission light can be ignored. The wavelength ranges of 650 nm to 850 nm and 1060 nm to 1090 nm are also ranges in which light absorption by the cells is sufficiently small as compared to the light scattering by the cells, and thus the effect of light absorption by the cells on the intensity of the emission light can be ignored. Accordingly, in the case of using the irradiation light having at least one wavelength included in the ranges of 650 nm to 850 nm and 1060 nm to 1090 nm for irradiating the cells, the parameters relating to the cell concentration can be accurately obtained on the basis of the amount of change in light intensity due solely to the scattering, and thus a state of the cell suspension can be accurately observed. With the above observation device, the parameters relating to the cell concentration can be obtained through a simple task of radiating the irradiation light onto the cell suspension in which the cells are suspended. According to this method, as compared to methods in which tasks of collecting and injecting the cell suspension into a device occur, the state of the cell suspension can be efficiently observed.
(2) In the observation device described in the above (1), the irradiation unit may include a light source using a semiconductor light emitting element that emits, as the irradiation light, light having one wavelength included in the ranges of 650 nm to 850 nm and 1060 nm to 1090 nm. In this case, by using the semiconductor light emitting element with a stable light intensity as the light source, the risk of fluctuations in the intensity of the emission light due to factors other than the state of the cell suspension (for example, instability in the intensity of the irradiation light) can be reduced. Thus, the state of the cell suspension can be observed more reliably based on the intensity of the emission light.
(3) In the observation device described in the above (1) or (2), the light receiving unit may be disposed at a position opposite to the irradiation unit with the cell suspension interposed therebetween, and may receive part of the irradiation light transmitted through the cell suspension as the emission light. In this case, the position of the light receiving unit relative to the irradiation unit can be easily adjusted.
(4) In the observation device described in any of the above (1) to (3), the irradiation unit may irradiate the cell suspension with first irradiation light having a first wavelength included in the ranges of 650 nm to 850 nm and 1060 nm to 1090 nm, and second irradiation light having a second wavelength that is included in the ranges of 650 nm to 850 nm and 1060 nm to 1090 nm and is different from the first wavelength. The light receiving unit may receive first emission light and second emission light, which have different wavelengths from each other, and which are emitted from the cell suspension as a result of radiating the first irradiation light and the second irradiation light. The analysis unit may determine the parameter relating to the cell concentration on the basis of an intensity of the first emission light and an intensity of the second emission light. The light scattering that occurs in the cells when the cell suspension is irradiated with light includes Rayleigh scattering by particles with sizes smaller than the wavelength of light, and Mie scattering by particles with sizes equal to or larger than the wavelength of light. Among these, Rayleigh scattering is primarily caused by molecules forming a cell membrane. If the cells in the cell suspension adhere to each other to form a cell cluster, an amount and a structure of the molecules forming the cell membrane of the cells in the cell cluster do not change, and thus the Rayleigh scattering intensity from the cell suspension does not change due to light irradiation. The Mie scattering intensity obtained from the cell suspension can change because the sizes of the particles (cell cluster) formed by cell adhesion can change. In this case, it is assumed that the intensity of light emitted from the cell suspension changes in response to changes in the Mie scattering intensity, even though the number of cells in the cell suspension has not changed. Such changes in light intensity can lead to less accurate estimation of a parameter relating to a cell concentration. Accordingly, if only Rayleigh scattering of the scattering that occurs in the cell suspension can be observed, the parameter relating to the cell concentration can be determined correctly without being affected by the adhesion between the cells. While the Rayleigh scattering intensity depends on the wavelength of light, the Mie scattering intensity does not depend on the wavelength of light, or does not depend significantly on the wavelength of light. For that reason, in the case of observing the state of the cell suspension using light of a plurality of wavelengths, by utilizing this difference in wavelength dependence, only the wavelength-dependent Rayleigh scattering intensity can be determined, and thus it is possible to accurately determine the parameter relating to the "cell concentration." The "Intensity of Rayleigh scattering" and the "Rayleigh scattering intensity" indicate an "amount of change in light intensity due to Rayleigh scattering," and the "intensity of Mie scattering" and the "Mie scattering intensity" indicate an "amount of change in light intensity due to Mie scattering."
(5) In the observation device described in the above (4), the irradiation unit may include a first light source that emits the first irradiation light, a second light source that emits the second irradiation light, and a switching unit that switches an optical path of the first irradiation light and an optical path of the second irradiation light so that the first irradiation light and the second irradiation light are selectively radiated onto the cell suspension. The light receiving unit may include an optical sensor that selectively receives the first emission light and the second emission light emitted from the cell suspension. In this case, the intensity of the first emission light and the intensity of the second emission light can be detected separately using one optical sensor. As a result, there is no need to separately prepare an optical sensor for detecting the intensity of the first emission light and an optical sensor for detecting the intensity of the second emission light, and thus complication of the configuration of the light receiving unit can be avoided.
(6) In the observation device described in the above (4), the irradiation unit may include a first light source that emits the first irradiation light, a second light source that emits the second irradiation light, and a combining unit that combines the first irradiation light and the second irradiation light to generate combined irradiation light and irradiate the cell suspension with the combined irradiation light. The light receiving unit may include a spectroscopic unit that spectrally separates the combined emission light emitted from the cell suspension as a result of radiating the combined irradiation light into respective wavelengths, a first optical sensor that detects the intensity of the first emission light spectrally separated from the combined emission light, and a second optical sensor that detects the intensity of the second emission light spectrally separated from the combined emission light. In this case, the intensity of the first emission light and the intensity of the second emission light can be detected simultaneously using the combined irradiation light and the combined emission light, so that observation of the state of the cell suspension based on the intensity of the first emission light and the second emission light can be performed more quickly and efficiently.
(7) In the observation device described in the above (4), the irradiation unit may include a light source that emits light including the first irradiation light and the second irradiation light, and a filter switching unit that includes a first filter configured to transmit only the first irradiation light, and a second filter configured to transmit only the second irradiation light, and that switches the positions of the first filter and the second filter so that the first filter or the second filter is disposed on an optical path of the light between the light source and the cell suspension. The light receiving unit may include an optical sensor that selectively receives the first and second emission lights emitted from the cell suspension. In this case, for example, by using an inexpensive lamp light source as the light source, the cost of the observation device can be reduced as compared to when an expensive laser light source is used as the light source.
(8) The observation method of the present disclosure is an observation method for observing a cell suspension including a plurality of cells. The observation method includes the steps of: irradiating a cell suspension with irradiation light having at least one wavelength included in the ranges of 650 nm to 850 nm and 1060 nm to 1090 nm; receiving emission light emitted from the cell suspension as a result of irradiating the cell suspension with irradiation light; and determining a parameter relating to a cell concentration of the cells in the cell suspension on the basis of an intensity of the emission light. According to this observation method, the same effect as the observation device described above can be obtained.
(9) In the observation method described in the above (8), in the step of irradiating the cell suspension, first irradiation light having a first wavelength included in the ranges of 650 nm to 850 nm and 1060 nm to 1090 nm, and second irradiation light having a second wavelength that is included in the ranges of 650 nm to 850 nm and 1060 nm to 1090 nm and is different from the first wavelength may be radiated to the cell suspension. In the step of receiving the emission light, the first emission light and the second emission light, which have different wavelengths from each other, and which are emitted from the cell suspension as a result of radiating the first irradiation light and the second irradiation light may be received. In the step of determining the parameter relating to the cell concentration, the parameter relating to the cell concentration may be determined on the basis of the intensity of the first emission light and the intensity of the second emission light. In this case, the same effects as the above (4) can be obtained.

### [Details of Embodiments of Present Disclosure]

Specific examples of an observation device and an observation method of the present disclosure will be described below with reference to the drawings. The present disclosure is not limited to these examples but is indicated by the scope of the claims, and is intended to include meanings equivalent to the scope of the claims and all modifications within the scope.

### First Embodiment

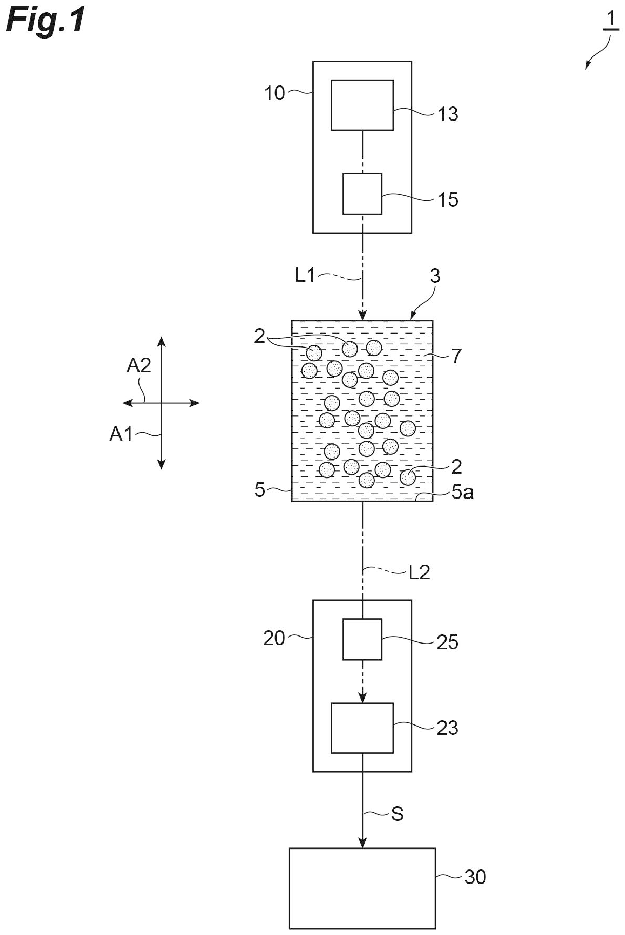

Fig. 1 is a diagram showing a configuration of an observation device 1 according to a first embodiment. The observation device 1 is a device for observing a state of a cell suspension 3. The cell suspension 3 contains a plurality of cells 2 and a culture medium 7 in which the plurality of cells 2 are suspended. Each cell 2 may be a cell collected from an animal or human, a stem cell collected from an animal or human, a stem cell created on the basis of a cell collected from an animal or human, or a cell differentiated from a stem cell. The cells 2 may contain a mixture of a plurality of cell types.

Some or all of the cells 2 contained in the cell suspension 3 may be dispersed discretely or aggregated with each other. That is, some or all of the cells 2 in the cell suspension 3 may be separated from each other or in contact with each other. Some or all of the cells 2 may form cell clusters (spheroids, organoids, assembloids, tissues, or organs) formed by aggregation with each other. A cell cluster is a cell accumulation in which a plurality of cells 2 are stacked in multiple layers. The cells 2 may be of a plurality of types. The cell clusters may include various forms, such as two-dimensional clusters and three-dimensional clusters. The cells 2 in the cell suspension 3 may fill a container 5 even when they are floating.

A "state" of the cell suspension 3 observed by the observation device 1 is represented by a parameter relating to a "cell concentration" of the cell suspension 3. The parameter relating to the "cell concentration" may be the "cell concentration" itself, or a "cell number" determined from the "cell concentration." The "cell number" is determined from the "cell concentration" and a volume of the container 5 of the cell suspension 3 or a volume of the culture medium 7. The volume of the container 5 is a constant, and the volume of the culture medium 7 can be known beforehand, and thus if the "cell concentration" can be determined, the "cell number" can also be determined. The parameter relating to the "cell concentration" may be a parameter indicating either the "cell concentration" or the "cell number," or may include both a parameter indicating the "cell concentration" and a parameter indicating the "cell number." In the present embodiment, the parameter relating to the "cell concentration" will be described as indicating the "cell concentration" itself. The "cell number" indicates the total number of the cells 2 contained in the cell suspension 3. The "cell concentration" indicates the number of cells 2 present in a unit volume of the cell suspension 3.

As shown in Fig. 1, the observation device 1 includes, for example, an irradiation unit 10, a light receiving unit 20, and an analysis unit 30. The irradiation unit 10 is disposed at a position facing the cell suspension 3. The light receiving unit 20 is disposed at a position opposite to the irradiation unit 10 with respect to the cell suspension 3. The cell suspension 3 is housed in the container 5. The container 5 is made of, for example, a transmissive material through which irradiation light L1 emitted from the irradiation unit 10 and emission light L2 incident on the light receiving unit 20 can be transmitted, such as a glass or resin material. The observation device 1 may include a placement stage on which the container 5 is placed. The transmissive material is, for example, a material that has a transmittance of 20% or more for light of 650 nm to 850 nm and 1060 nm to 1090 nm when the material is 1 mm thick.

For example, each cell 2 is suspended inside the container 5 while immersed in the culture medium 7. For example, each cell 2 may be obtained by culturing cells while attaching them to a scaffold material containing particles such as resin spheres, or by culturing cells while suspending them in a culture medium in the absence of a scaffold material. Accordingly, the cell suspension 3 may contain particles such as resin spheres as the scaffold material for the cells 2. Hereafter, a first direction in a vertical direction A1 along a normal to a bottom surface 5a of the container 5 may be referred to as "upward," and a second direction opposite to the first direction may be referred to as "downward." A direction perpendicular to the vertical direction A1 will be referred to as a lateral direction A2.

The irradiation unit 10 includes, for example, a light source 13 and a lens 15. The light source 13 is disposed above the cell suspension 3 and is facing the cell suspension 3 in the vertical direction A1. The light source 13 emits the irradiation light L1 toward an irradiation region set in the cell suspension 3. The irradiation region is a region forming part of the cell suspension 3 when viewed from above, and is a region irradiated with the irradiation light L1 from the light source 13. The irradiation region may be, for example, a central portion of the cell suspension 3 when viewed from above. An optical axis of the irradiation light L1 emitted from the light source 13 is directed in the vertical direction A1. In one example, the optical axis of the irradiation light L1 is, for example, perpendicular to the bottom surface 5a and parallel to the vertical direction A1. The light source 13 is, for example, a laser light source using a laser diode (LD). The irradiation light L1 has one wavelength included in the ranges of 650 nm to 850 nm and 1060 nm to 1090 nm. The ranges of 650 nm to 850 nm and 1060 nm to 1090 nm indicate ranges that include both the range of 650 nm to 850 nm and the range of 1060 nm to 1090 nm. Accordingly, it can be said that the irradiation light L1 has one wavelength included in at least one of the range of 650 nm to 850 nm and the range of 1060 nm to 1090 nm.

The light source 13 is not limited to a laser light source using a laser diode. For example, the light source 13 may be a light source using a semiconductor light emitting element such as a laser diode, a light emitting diode (LED), or a super luminescent diode (SLD), a laser light source using an element other than a semiconductor light emitting element, or a lamp light source. For example, if a lamp light source is used, the irradiation light L1 may have, in addition to a wavelength included in the ranges of 650 nm to 850 nm and 1060 nm to 1090 nm, one or more wavelengths different from that wavelength. In this case, one or more wavelengths different from that wavelength may be wavelengths included in the ranges of 650 nm to 850 nm and 1060 nm to 1090 nm, or may be wavelengths outside the ranges of 650 nm to 850 nm and 1060 nm to 1090 nm.

The lens 15 is disposed on an optical path of the irradiation light L1 between the light source 13 and the cell suspension 3. The lens 15 is, for example, a condensing lens that condenses the irradiation light L1 from the light source 13. The lens 15 may be a collimating lens that collimates the irradiation light L1. The irradiation light L1 emitted from the light source 13 is radiated through the lens 15 to the cell suspension 3. Part of the irradiation light L1 is transmitted through the cell suspension 3 and is emitted as the emission light L2. In the present embodiment, the emission light L2 is transmitted light of the irradiation light L1 that has been transmitted through the cell suspension 3.

The light receiving unit 20 includes, for example, an optical sensor 23 and a lens 25. The optical sensor 23 is disposed below the cell suspension 3 and faces the light source 13 in the vertical direction A1 with the cell suspension 3 interposed therebetween. The optical sensor 23 is disposed at a position opposite to the light source 13 in the vertical direction A1 with the cell suspension 3 interposed therebetween. The optical sensor 23 is disposed on an optical path of the emission light L2 emitted from the cell suspension 3, that is, on an extension line of the optical axis of the irradiation light L1. The optical sensor 23 receives the emission light L2 emitted from the cell suspension 3. An optical axis of the emission light L2 is, for example, perpendicular to the bottom surface 5a on which the cell suspension 3 is placed, and parallel to the vertical direction A1.

The lens 25 is disposed on the optical path of the emission light L2 between the cell suspension 3 and the optical sensor 23. The lens 25 is, for example, an objective lens. The lens 25 may be a collimating lens. The emission light L2 emitted from the cell suspension 3 passes through the lens 25 and is incident on the optical sensor 23. The optical sensor 23 receives the emission light L2 and outputs an electric signal S corresponding to the intensity of the emission light L2 to the analysis unit 30. If the irradiation light L1 includes a plurality of wavelengths, a filter that transmits light of a specific wavelength may be disposed at any position between the light source 13 and the optical sensor 23.

As shown in Fig. 1, the analysis unit 30 is communicatively connected to the light receiving unit 20. The analysis unit 30 may be communicatively connected to the irradiation unit 10. The analysis unit 30 receives the electric signal S from the light receiving unit 20. The analysis unit 30 determines the "cell concentration" of the cell suspension 3 on the basis of the received electric signal S. The analysis unit 30 may determine the "cell number" obtained from the "cell concentration." The intensity of the emission light L2 indicated by the electric signal S directly correlates with the "cell concentration." For that reason, the analysis unit 30 may determine only the "cell concentration" on the basis of the electric signal S, or may determine the "cell number" by using the volume of the container 5 as a constant or by acquiring the volume of the culture medium 7 beforehand. The analysis unit 30 may determine both the "cell concentration" and the "cell number," or either the "cell concentration" or the "cell number."

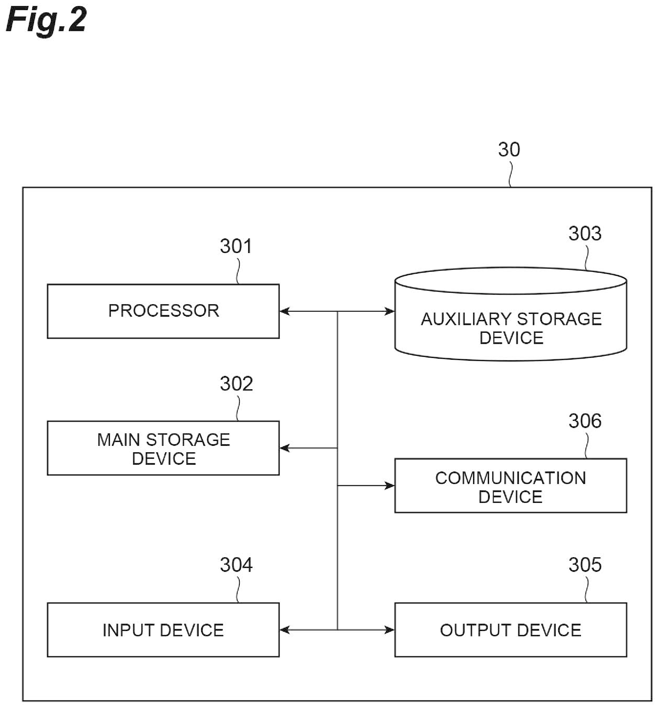

Fig. 2 is a diagram showing a hardware configuration of the analysis unit 30. The analysis unit 30 is physically one or more computers. As shown in Fig. 2, the analysis unit 30 physically includes hardware such as one or more processors 301, a main storage device 302, an auxiliary storage device 303, an input device 304, an output device 305, and a communication device 306. The analysis unit 30 is constituted by one or more physical computers configured with the physical hardware and software such as programs.

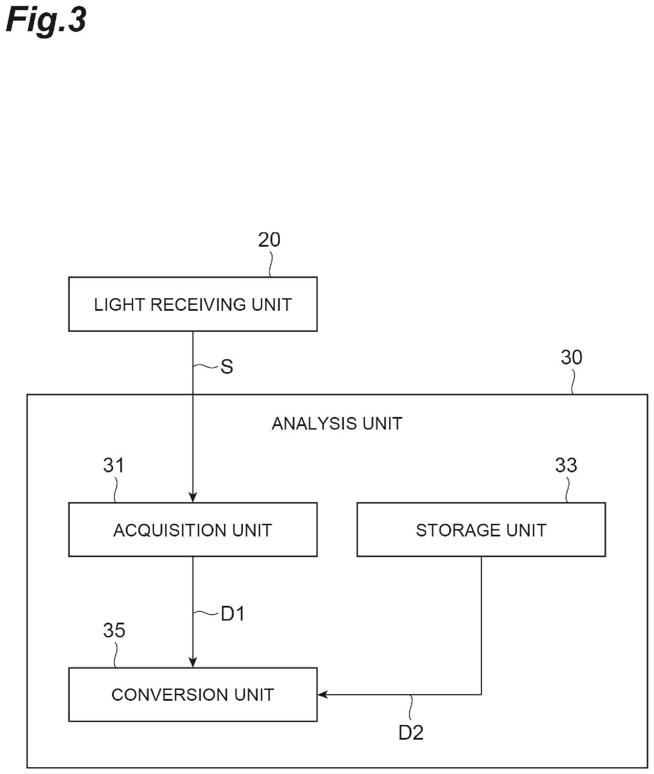

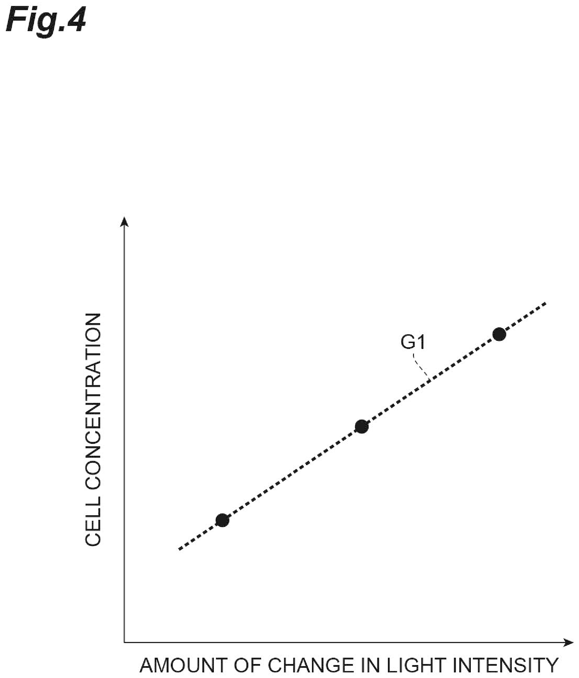

Fig. 3 is a diagram showing a functional configuration of the analysis unit 30. Fig. 4 is a graph showing a calibration curve G1 held by the analysis unit 30. The analysis unit 30 includes, as functional components, an acquisition unit 31, a storage unit 33, and a conversion unit 35, for example. Each functional component of the analysis unit 30 is implemented by executing a program on the hardware of the physical computer described above.

The acquisition unit 31 acquires the intensity of the emission light L2 indicated by the electric signal S. The acquisition unit 31 provides light intensity information D1 indicating the intensity of the emission light L2 to the conversion unit 35.

The storage unit 33 stores conversion information D2 for determining the "cell concentration" of the cell suspension 3 on the basis of the intensity of the emission light L2. The conversion information D2 includes, for example, the calibration curve G1 showing the relationship between the amount of change in light intensity obtained from the light intensity information D1 and the "cell concentration" of the cell suspension 3. As shown in Fig. 4, the calibration curve G1 is a graph plotting the relationship between the amount of change in light intensity and the "cell concentration" of the cell suspension 3. The amount of change in light intensity is a relative ratio between the intensity of the irradiation light L1 and the intensity of the emission light L2, and indicates an amount of change in the intensity of the emission light L2 relative to the intensity of the irradiation light L1. The amount of change in light intensity is defined based on the intensity of the emission light L2 divided by the intensity of the irradiation light L1 (-10Log(intensity of emission light L2/intensity of irradiation light L1)).

As shown in Fig. 4, the amount of change in light intensity increases as the "cell concentration" increases and decreases as the "cell concentration" decreases. For that reason, if a magnitude of the amount of change in light intensity is known, the "cell concentration" can be estimated. By preparing beforehand a sample of the cell suspension 3 with a known "cell concentration" (or a sample having a "cell concentration" serving as a reference) and irradiating the sample with the irradiation light L1 using the observation device 1, the calibration curve G1 showing the relationship between the amount of change in light intensity and the "cell concentration" of the sample can be created in advance.

As shown in Fig. 3, the storage unit 33 stores beforehand the calibration curve G1 thus created in advance, and provides the conversion information D2 including the calibration curve G1 to the conversion unit 35. The conversion information D2 may include, instead of or together with the calibration curve G1, a conversion formula (conversion equation) showing the relationship between the amount of change in light intensity and the "cell concentration." The conversion information D2 may include, in addition to the calibration curve G1, information required for converting the light intensity information D1 to the "cell concentration." For example, the conversion information D2 may include the intensity of the irradiation light L1 for determining the amount of change in light intensity from the light intensity information D1, or may include the volume of the container 5 or the volume of the culture medium 7 required for determining the "cell number" from the "cell concentration."

The conversion unit 35 determines the "cell concentration" of the cell suspension 3 using the light intensity information D1 and the conversion information D2. First, the conversion unit 35 calculates the amount of change in light intensity, which is the relative ratio between the intensity of the emission light L2 and the intensity of the irradiation light L1. Next, the conversion unit 35 converts the calculated amount of change in light intensity into the "cell concentration" of the cell suspension 3 by referring to the calibration curve G1. The analysis unit 30 may output the observation results, including the "cell concentration" of the cell suspension 3 determined by the conversion unit 35, using the output device 305, such as a monitor or printer, for example.

In the observation device 1 described above, the irradiation light L1 is radiated onto a partial irradiation region of the cell suspension 3, and thus, in a strict sense, the intensity of the emission light L2 from the irradiation region depends on the "cell concentration" (or the "cell number") in that irradiation region. Since the cells 2 in the cell suspension 3 are usually uniformly distributed, the same intensity of the emission light L2 is obtained regardless of which irradiation region of the cell suspension 3 is irradiated with the irradiation light L1. Accordingly, in order to determine the "cell concentration" of the cell suspension 3, it is not needed to irradiate a plurality of regions of the cell suspension 3 with the irradiation light L1, and it is sufficient to irradiate a partial region of the cell suspension 3 with the irradiation light L1. However, if a distribution of the cells 2 in the cell suspension 3 is not uniform, a plurality of regions of the cell suspension 3 may be irradiated with the irradiation light L1.

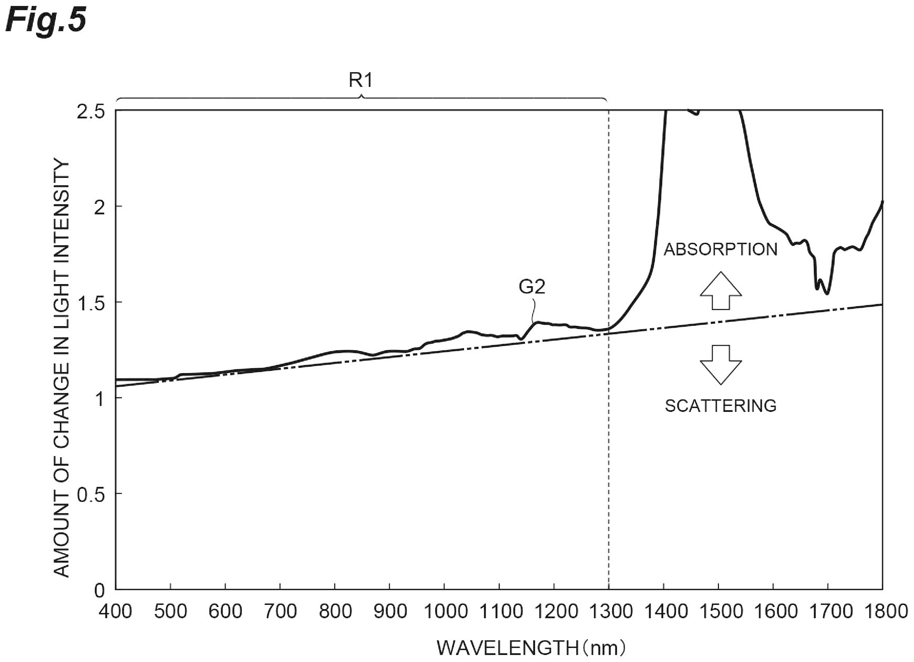

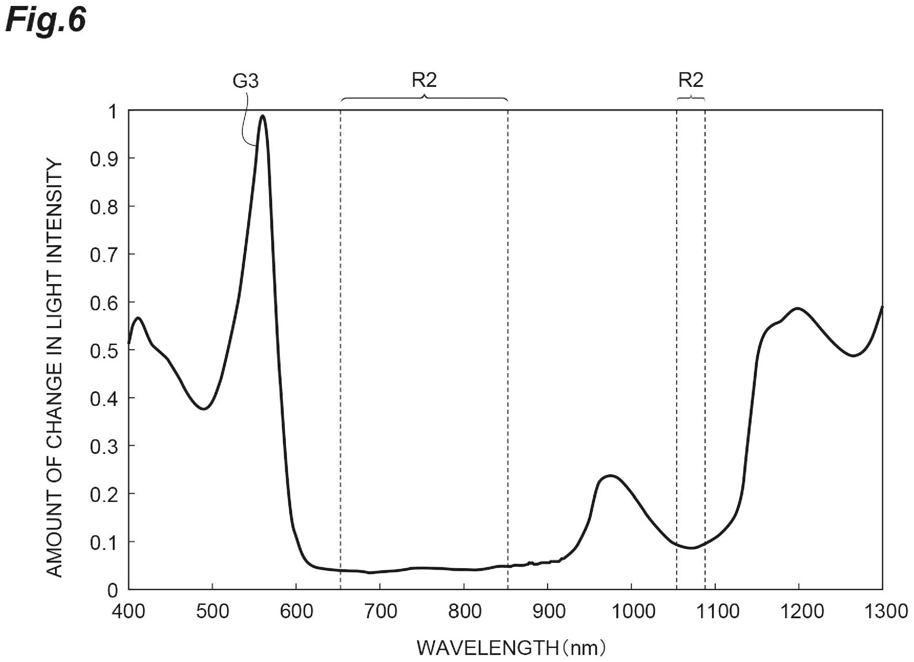

With reference to Figs. 5 and 6, the reason for using the irradiation light L1 in the wavelength ranges of 650 nm to 850 nm and 1060 nm to 1090 nm for irradiating the cell suspension 3 will be described below.

Fig. 5 is a graph showing a spectrum of an amount of change in light intensity of the cells 2. The vertical axis in Fig. 5 represents the amount of change in light intensity of the cells 2, and the horizontal axis represents wavelength [nm]. The vertical axis in Fig. 5 can also be said to represent scattering and absorption intensities of the cells 2. Fig. 6 is a graph showing a spectrum of an amount of change in light intensity of the culture medium 7. The vertical axis in Fig. 6 represents the amount of change in light intensity of the culture medium 7, and the horizontal axis represents wavelength [nm]. Since no scattering occurs in the culture medium 7, the vertical axis in Fig. 6 can also be said to represent the absorption intensity of the culture medium 7.

When light is radiated onto the cell suspension 3, light absorption by the cells 2 and the culture medium 7 occurs, and light scattering by the cells 2 occurs. A mixture of a plurality of phenomena such as light scattering and light absorption is reflected in the intensity of light emitted from the cell suspension 3 as a result of irradiating the cell suspension 3 with the light. The amount of change in light intensity due to the scattering among these phenomena depends on parameters relating to the "cell concentration" of the cell suspension 3. Accordingly, if the amount of change in light intensity due solely to the scattering can be acquired, it is possible to quantitatively determine the "cell concentration" of the cell suspension 3.

As shown in graph G2 of Fig. 5, in the wavelength range of 1300 nm or greater, there are many peaks in which the amount of change in light intensity of the cells 2 changes sharply. These peaks are caused by light absorption by the cells 2. Accordingly, in the wavelength range in which there are many peaks, light absorption has a significant effect on the intensity of the light emitted from the cell suspension 3, and thus it is difficult to obtain the amount of change in light intensity due solely to the scattering. In a wavelength range R1 of 1300 nm or less, it can be seen that there is no peak and the amount of change in light intensity changes extremely gradually. In such a wavelength range R1, the effect of light absorption on the intensity of the light emitted from the cell suspension 3 is sufficiently small and negligible as compared to the effect of scattering, and thus the amount of change in light intensity due solely to the scattering can be easily obtained.

As shown in graph G3 of Fig. 6, within the wavelength range R1 of 1300 nm or less (see Fig. 5), in the wavelength ranges R2 of 650 nm to 850 nm and 1060 nm to 1090 nm, it can be seen that the amount of change in light intensity due solely to light absorption by the culture medium 7 is sufficiently smaller than the amount of change in light intensity due to light scattering by the cells 2 shown in Fig. 5. Accordingly, if the cell suspension 3 is irradiated with light in the wavelength range R2 of 650 nm to 850 nm and 1060 nm to 1090 nm, the effect of light absorption by the culture medium 7 can be ignored. That is, the risk of light absorption by the culture medium 7 being reflected in the intensity of the light emitted from the cell suspension 3 can be reduced. The culture medium 7 may contain water, amino acids, vitamins, salts, and glucose. An indicator such as phenol red may be added to the culture medium 7, for example. If the effect of light absorption by the culture medium 7 can be ignored, it is possible to obtain the amount of change in light intensity that reflects only scattering by the cell suspension 3. Ultraviolet light with a wavelength shorter than 400 nm is not appropriate for irradiating the cell suspension 3 because it may damage the cells 2.

Accordingly, in the case of using the irradiation light L1 having at least one wavelength included in the wavelength ranges R2 of 650 nm to 850 nm and 1060 nm to 1090 nm for irradiating the cell suspension 3, the effect of absorption of the irradiation light L1 by the cells 2 and the culture medium 7 can be ignored, as described above, and thus it is possible to obtain the intensity of the emission light L2 that reflects only the scattering of the irradiation light L1 in the cell suspension 3. As a result, it is possible to quantitatively determine the "cell concentration" of the cell suspension 3 on the basis of the intensity of the emission light L2 obtained. In this way, in the present embodiment, on the basis of the spectrum of the amount of change in light intensity of the cells 2 and the spectrum of the amount of change in light intensity of the culture medium 7, the wavelength ranges R2 of 650 nm to 850 nm and 1060 nm to 1090 nm are selected as wavelength ranges in which the effect of absorption of the irradiation light L1 by the cell suspension 3 and the culture medium 7 can be ignored.

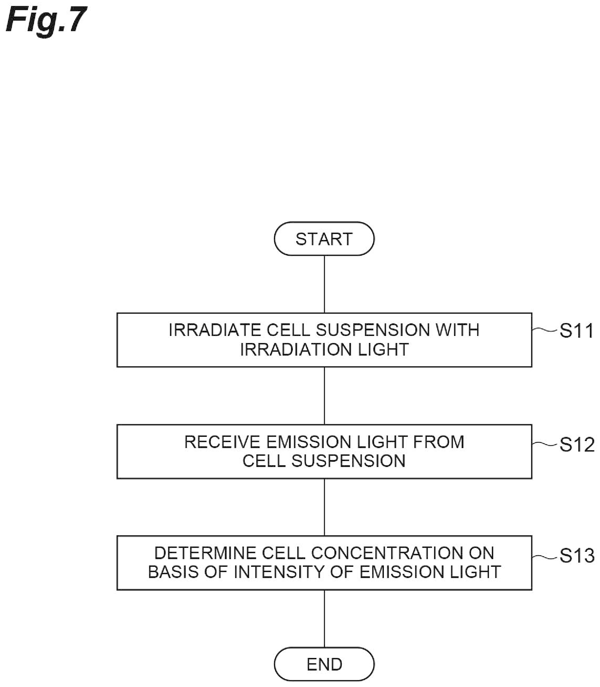

Steps of the observation method implemented using the observation device 1 described above will be described with reference to Fig. 7. Fig. 7 is a flowchart showing an example of each step of the observation method.

First, the irradiation unit 10 disposed above the cell suspension 3 irradiates the cell suspension 3 with the irradiation light L1 at least one wavelength included in the wavelength ranges of 650 nm to 850 nm and 1060 nm to 1090 nm (step S11). Part of the irradiation light L1 radiated to the cell suspension 3 is transmitted through the cell suspension 3 and emitted from the cell suspension 3 as the emission light L2.

Next, the light receiving unit 20 disposed below the cell suspension 3 receives the emission light L2 emitted from the cell suspension 3 (step S12). The light receiving unit 20 outputs the electric signal S corresponding to the intensity of the received emission light L2 to the analysis unit 30.

Next, the analysis unit 30 determines the "cell concentration" of the cell suspension 3 on the basis of the intensity of the emission light L2 indicated by the electric signal S (step S13). Specifically, the conversion unit 35 of the analysis unit 30 calculates the amount of change in light intensity by dividing the intensity of the emission light L2 by the intensity of the irradiation light L1. Then, the conversion unit 35 converts the calculated amount of change in light intensity into the "cell concentration" of the cell suspension 3 by referring to the calibration curve G1 shown in Fig. 4. Using the "cell concentration" determined in this way, it is possible to quantitatively observe the state of the cell suspension 3.

Effects obtained by the first embodiment described above will be described. As described above, in the spectrum of the amount of change in light intensity of the culture medium 7, the wavelength ranges R2 of 650 nm to 850 nm and 1060 nm to 1090 nm (see Fig. 6) are ranges in which light absorption by the culture medium 7 is sufficiently smaller than light scattering by the cells 2 shown in Fig. 5. Therefore, in the case of using the irradiation light L1 having at least one wavelength included in the wavelength ranges R2 of 650 nm to 850 nm and 1060 nm to 1090 nm for irradiating the cell suspension 3, the effect of light absorption by the culture medium 7 on the intensity of the emission light L2 can be ignored. In the spectrum of the amount of change in light intensity of the cells 2, the wavelength ranges R2 of 650 nm to 850 nm and 1060 nm to 1090 nm are included in the wavelength range R1 (see Fig. 5), in which there are no sharp peaks caused by light absorption by the cells 2, and thus the effect of light absorption by the cell suspension 3 on the intensity of the emission light L2 is sufficiently smaller than the scattering occurring in the cells 2 and can be ignored.

Accordingly, in the case of using the irradiation light L1 having at least one wavelength in the wavelength ranges R2 of 650 nm to 850 nm and 1060 nm to 1090 nm for irradiating the cell suspension 3, the intensity of the emission light L2 due solely to the scattering can be acquired, and thus the "cell concentration" of the cell suspension 3 can be accurately determined. In the present embodiment, the "cell concentration" of the cell suspension 3 can be determined by a simple task of irradiating the cell suspension 3 with the irradiation light L1 while the cells 2 are suspended in the culture medium 7. According to this method, as compared to methods in which tasks of collecting and injecting the cell suspension 3 into a device occur, the state of the cell suspension 3 can be observed efficiently.

As in the present embodiment, the irradiation unit 10 may include the light source 13 using a laser diode that emits light having one wavelength included in the ranges of 650 nm to 850 nm and 1060 nm to 1090 nm as the irradiation light L1. The light source 13 may be a light source using a semiconductor light emitting element such as a laser diode, a light emitting diode diode, or a super luminescent diode. In this case, by using light from the light source using a semiconductor light emitting element with a stable light intensity as the irradiation light L1, the risk of fluctuations in the intensity of the emission light L2 due to factors other than the state of the cell suspension 3 (for example, instability in the intensity of the irradiation light) can be reduced. Thus, the state of the cell suspension 3 can be observed more reliably on the basis of the intensity of the emission light L2.

As in the present embodiment, the light receiving unit 20 may be disposed at a position opposite to the irradiation unit 10 with the cell suspension 3 interposed therebetween, and may receive part of the irradiation light L1 transmitted through the cell suspension 3 as the emission light L2. In this case, the position of the light receiving unit 20 relative to the irradiation unit 10 can be easily adjusted.

The observation device and the observation method of the present disclosure are not limited to the first embodiment described above, and various other modifications are possible.

### <Modified Example 1>

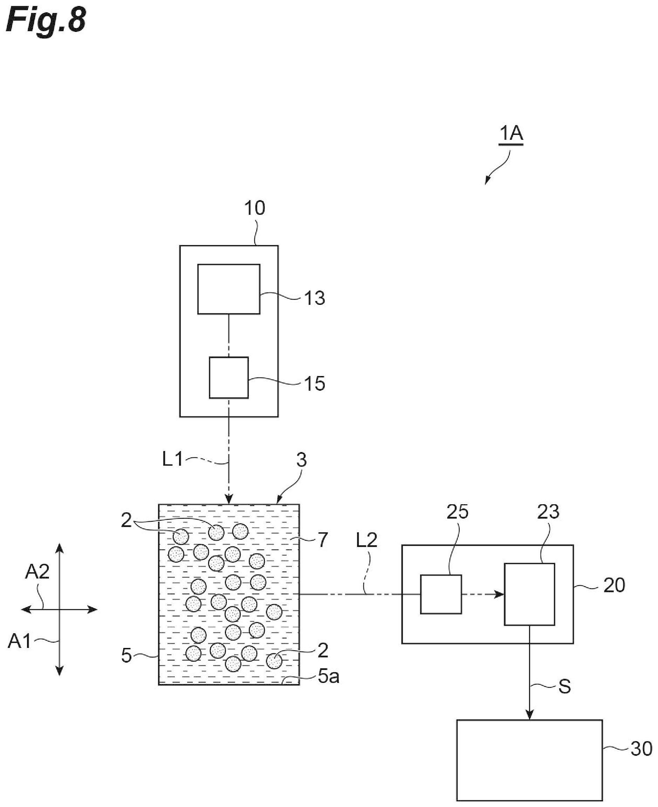

Fig. 8 is a diagram showing a modified example of the observation device 1. As in an observation device 1A shown in Fig. 8, the light receiving unit 20 may be disposed at a position facing the cell suspension 3 in the lateral direction A2. In this case, the light receiving unit 20 receives the emission light L2 emitted from the cell suspension 3 in the lateral direction A2 as a result of radiating the irradiation light L1 from the light source 13 of the irradiation unit 10. In this case, the emission light L2 is scattered light of the irradiation light L1 scattered in the lateral direction A2 in the cell suspension 3. The light receiving unit 20 is disposed on the optical path of the emission light L2 extending from the cell suspension 3 in the lateral direction A2. The light receiving unit 20 receives the emission light L2 through the lens 25 and outputs the electric signal S indicating the intensity of the emission light L2 to the analysis unit 30. The optical axis of the emission light L2 is, for example, parallel to the lateral direction A2 and perpendicular to the vertical direction A1 along which the irradiation light L1 extends.

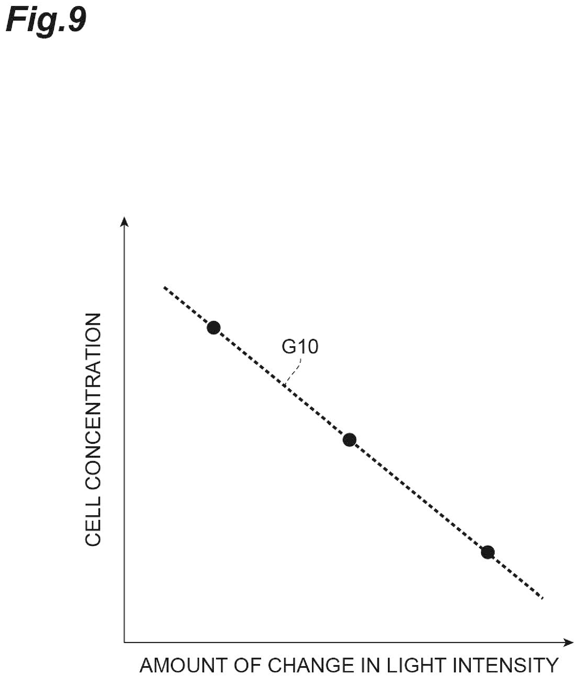

Fig. 9 is a graph showing a calibration curve G10 held by the analysis unit 30. The calibration curve G10 is a graph plotting the relationship between the amount of change in light intensity and the "cell concentration" of the cell suspension 3. The amount of change in light intensity is a relative ratio between the intensity of the irradiation light L1 and the intensity of the emission light L2, and indicates an amount of change in the intensity of the emission light L2 relative to the intensity of the irradiation light L1. The amount of change in light intensity is defined based on a value obtained by dividing the intensity of the emission light L2 by the intensity of the irradiation light L1 (-10Log(intensity of emission light L2/intensity of irradiation light L1)). As shown in Fig. 9, the amount of change in light intensity decreases as the "cell concentration" increases, and increases as the "cell concentration" decreases. For that reason, if a magnitude of the amount of change in light intensity is known, the "cell concentration" can be estimated.

The analysis unit 30 calculates the amount of change in light intensity, which is the relative ratio between the intensity of the emission light L2 and the intensity of the irradiation light L1, and then converts the calculated amount of change in light intensity into the "cell concentration" of the cell suspension 3 by referring to the calibration curve G10. Since the observation device 1A shown in Fig. 8 can also quantitatively determine the "cell concentration" on the basis of the intensity of the emission light L2 from the cell suspension 3, the same effects as the first embodiment described above can be obtained.

### <Modified Example 2>

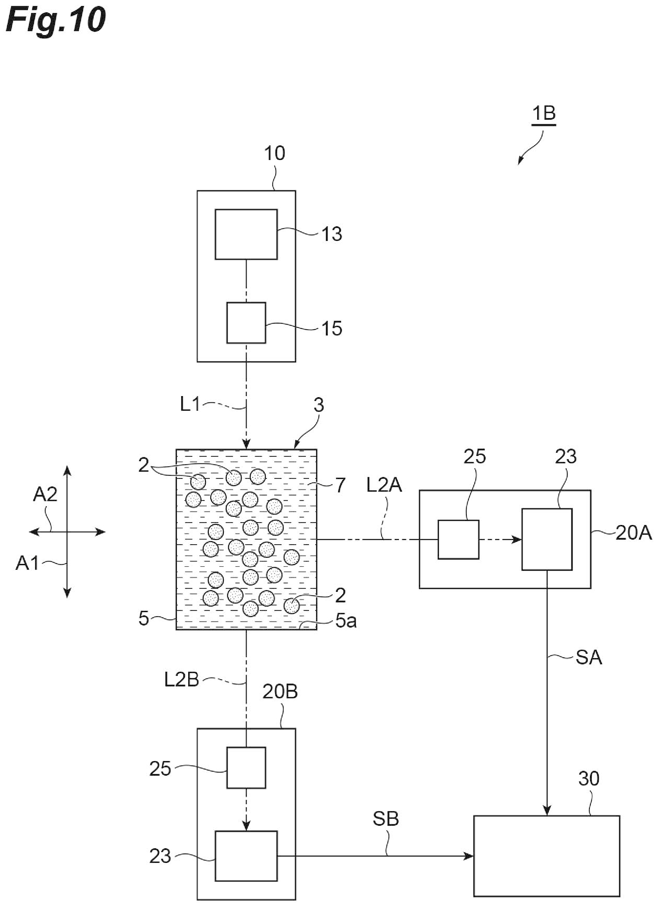

Fig. 10 is a diagram showing a modified example of the observation device 1. As shown in Fig. 10, an observation device 1B may include a first light receiving unit 20A and a second light receiving unit 20B. The first light receiving unit 20A has the same configuration as the light receiving unit 20 shown in Fig. 8. That is, the first light receiving unit 20A is disposed at a position facing the cell suspension 3 in the lateral direction A2 and receives, as first emission light L2A, scattered light scattered by the cell suspension 3 from the irradiation light L1 irradiated to the cell suspension 3. The first light receiving unit 20A outputs a first electric signal SA indicating an intensity of the first emission light L2A to the analysis unit 30.

The second light receiving unit 20B has the same configuration as the light receiving unit 20 of the first embodiment described above. That is, the second light receiving unit 20B is disposed at a position facing the irradiation unit 10 in the vertical direction A1 with the cell suspension 3 interposed therebetween and receives, as second emission light L2B, transmitted light that has been transmitted through the cell suspension 3 from the irradiation light L1 irradiated to the cell suspension 3. The second light receiving unit 20B outputs a second electric signal SB indicating an intensity of the second emission light L2B to the analysis unit 30.

The analysis unit 30 determines the "cell concentration" of the cell suspension 3 on the basis of the intensity of the first emission light L2A and the intensity of the second emission light L2B. For example, the analysis unit 30 converts a first amount of change in light intensity obtained from the intensity of the first emission light L2A into the "cell concentration" by referring to the calibration curve G10 shown in Fig. 9. The analysis unit 30 converts a second amount of change in light intensity obtained from the intensity of the second emission light L2B into the "cell concentration" by referring to the calibration curve G1 shown in Fig. 4. The analysis unit 30 may determine a more accurate "cell concentration" by averaging the "cell concentration" obtained on the basis of the intensity of the first emission light L2A and the "cell concentration" obtained on the basis of the intensity of the second emission light L2B.

Since the observation device 1B shown in Fig. 10 can also quantitatively determine the "cell concentration" on the basis of the intensity of the first emission light L2A and the intensity of the second emission light L2B, the same effects as those of the first embodiment described above can be obtained. By acquiring both the intensity of the first emission light L2A and the intensity of the second emission light L2B, the observation device 1B can more accurately determine the "cell concentration" on the basis of these intensities.

### Second Embodiment

An observation device according to a second embodiment will be described. In the following description of the second embodiment, descriptions of parts that overlap those of the first embodiment will be omitted as appropriate, and different parts from those of the first embodiment will be mainly described.

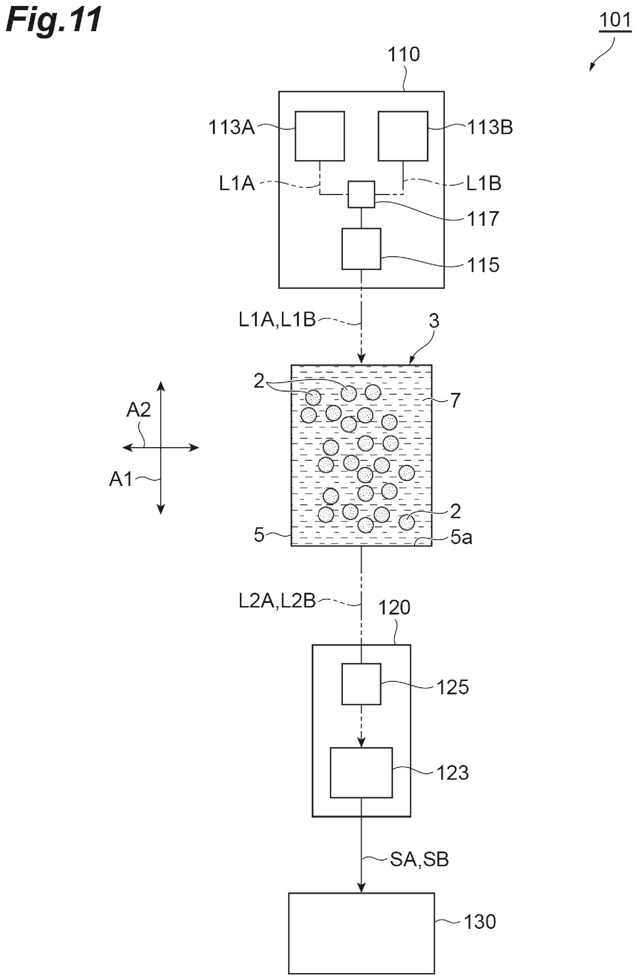

Fig. 11 is a diagram showing a configuration of an observation device 101 of the second embodiment. The observation device 101 in the present embodiment includes, for example, an irradiation unit 110, a light receiving unit 120, and an analysis unit 130. The light receiving unit 120 has the same configuration as the light receiving unit 20 of the first embodiment, for example. That is, as shown in Fig. 11, the light receiving unit 120 includes a lens 125 having the same configuration as the lens 25, and an optical sensor 123 having the same configuration as the optical sensor 23.

The irradiation unit 110 has a different configuration from the irradiation unit 10 of the first embodiment. The irradiation unit 110 includes a first light source 113A, a second light source 113B, a lens 115, and a switching unit 117. The lens 115 has the same configuration as the lens 15 in the first embodiment. The first light source 113A and the second light source 113B are disposed above the cell suspension 3 and adjacent to each other in the lateral direction A2. Each of the first light source 113A and the second light source 113B is a laser light source using a laser diode (LD), for example. Each of the first light source 113A and the second light source 113B may be a light source using a semiconductor light emitting element such as a laser diode, a light emitting diode (LED), or a super luminescent diode (SLD), or may be a laser light source using an element other than a semiconductor light emitting element, or a lamp light source.

The first light source 113A emits first irradiation light L1A having a first wavelength included in the ranges of 650 nm to 850 nm and 1060 nm to 1090 nm. The second light source 113B emits second irradiation light L1B having a second wavelength different from the first wavelength. The second wavelength is, for example, a wavelength different from the first wavelength in the ranges of 650 nm to 850 nm and 1060 nm to 1090 nm. Each of the first irradiation light L1A and the second irradiation light L1B may have a plurality of wavelengths. That is, the first irradiation light L1A may have other wavelengths different from the first wavelength, and the second irradiation light L1B may have other wavelengths different from the second wavelength.

The switching unit 117 is disposed between the first and second light sources 113A and 113B and the lens 115. The switching unit 117 is optically coupled to the first light source 113A, the second light source 113B, and the lens 115 using lenses and optical fibers. The switching unit 117 switches between an optical path of the first irradiation light L1A and an optical path of the second irradiation light L1B so that the first irradiation light L1A from the first light source 113A and the second irradiation light L1B from the second light source 113B are selectively radiated to the cell suspension 3. For example, the switching unit 117 sequentially irradiates the cell suspension 3 with the first irradiation light L1A and the second irradiation light L1B. The switching unit 117 is, for example, an optical switch that changes a traveling direction of light by sliding a mirror or prism. The switching unit 117 may be, for example, an optical switch that switches a traveling direction of light using a dielectric material with an electro-optic effect, such as LiON3 (lithium nitrate) or LiTaO3 (lithium tantalate).

When the cell suspension 3 is irradiated with the first irradiation light L1A, part of the first irradiation light L1A is transmitted through the cell suspension 3 and is emitted from the cell suspension 3 as the first emission light L2A. The first emission light L2A has the same wavelength as the first wavelength of the first irradiation light L1A. When the cell suspension 3 is irradiated with the second irradiation light L1B, part of the second irradiation light L1B is transmitted through the cell suspension 3 and is emitted from the cell suspension 3 as the second emission light L2B. The second emission light L2B has the same wavelength as the second wavelength of the second irradiation light L1B.

The optical sensor 123 selectively receives the first emission light L2A and the second emission light L2B emitted from the cell suspension 3. For example, the optical sensor 123 receives the first emission light L2A and the second emission light L2B in sequence. The optical sensor 123 outputs, to the analysis unit 130, the first electric signal SA corresponding to the intensity of the first emission light L2A and the second electric signal SB corresponding to the intensity of the second emission light L2B.

The analysis unit 130 quantitatively determines the "cell concentration" of the cell suspension 3 on the basis of the intensity of the first emission light L2A and the intensity of the second emission light L2B. In general, when light is radiated to the cell suspension 3, light absorption by the cells 2 and the culture medium 7 occurs, and light scattering by the cells 2 occurs. Among these phenomena, light scattering that occurs in the cells 2 includes Rayleigh scattering by particles with sizes smaller than the wavelength of light, and Mie scattering by particles with sizes equal to or larger than the wavelength of light. Since the size of the cells 2 is about 10 µm, Mie scattering occurs when light hits the cells 2. Rayleigh scattering occurs when light hits the molecules forming cell membranes of the cells 2.

The Rayleigh scattering intensity generated due to the internal molecular structure of the cells 2 in this way depends on parameters relating to the "cell concentration," such as the "cell concentration" and the "cell number" of the cell suspension 3. Suppose that the cells 2 in the cell suspension 3 adhere to each other and form a cell cluster. In this case, an amount and a structure of molecules forming the cell membrane of the cells 2 in the cell cluster do not change, and thus the Rayleigh scattering intensity obtained from the cell suspension 3 as a result of light irradiation does not change. The sizes of the particles (cell cluster) formed by the adhesion of the cells 2 to each other change, and thus the Mie scattering intensity obtained from the cell suspension 3 changes.

Accordingly, even if the same number of cells 2 are present in the cell suspension 3, the Rayleigh scattering intensity does not change but the Mie scattering intensity changes between a case in which the cells 2 are present while they adhere to each other and a case in which the cells 2 are present while they are separated from each other without adhesion. Therefore, estimating the "cell concentration" of the cell suspension 3 on the basis of a scattering intensity in which Rayleigh scattering and Mie scattering are mixed may result in an inaccurate "cell concentration." Resin spheres that can be used as a scaffold material for culturing the cells 2 have a homogeneous molecular structure, and thus when light hits such resin spheres, only Mie scattering occurs. Even in the presence of such a scaffold material, the "cell concentration" obtained on the basis of the scattering intensity may vary depending on the Mie scattering intensity.

Accordingly, if only Rayleigh scattering can be observed among scatterings occurring in the cell suspension 3, the "cell concentration" can be accurately estimated without being affected by the adhesion between the cells 2 or by the scaffold material. While the Rayleigh scattering intensity depends on the wavelength of light, the Mie scattering intensity does not depend on the wavelength of light, or does not depend significantly on the wavelength of light. For that reason, in the case of observing the state of the cell suspension 3 using light of a plurality of wavelengths, only the wavelength-dependent Rayleigh scattering intensity can be determined using the difference in wavelength dependence.

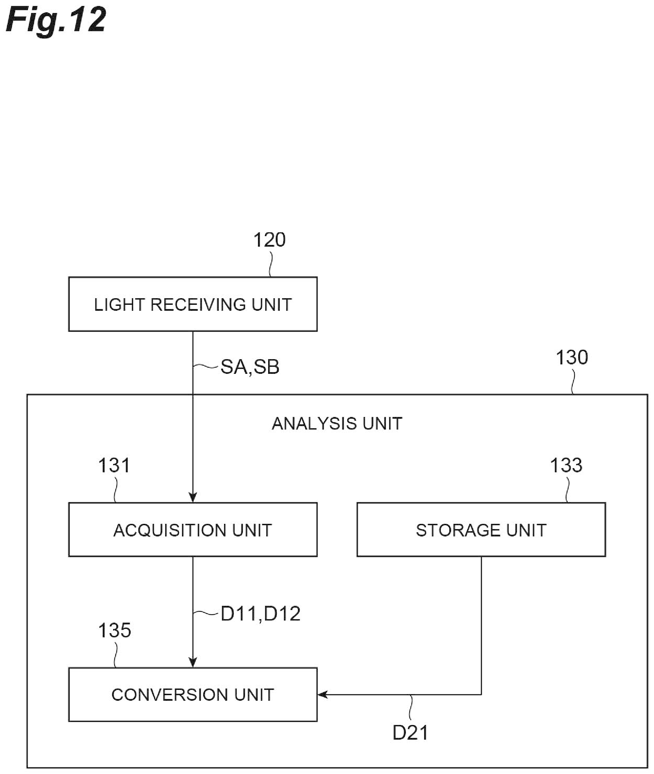

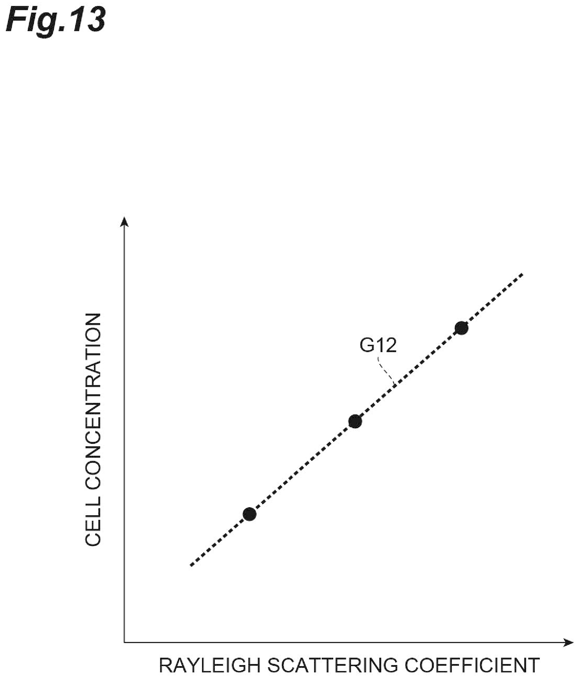

Fig. 12 is a diagram showing a functional configuration of the analysis unit 130. Fig. 13 is a graph showing a calibration curve G12 held by the analysis unit 130. The analysis unit 130 includes, as functional components, an acquisition unit 131, a storage unit 133, and a conversion unit 135, for example.

The acquisition unit 131 acquires the intensity of the first emission light L2A indicated by the first electric signal SA and the intensity of the second emission light L2B indicated by the second electric signal SB. The acquisition unit 131 provides the conversion unit 135 with first light intensity information D11 indicating the intensity of the first emission light L2A, and second light intensity information D12 indicating the intensity of the second emission light L2B.

The storage unit 133 stores conversion information D21 for determining the "cell concentration" of the cell suspension 3 on the basis of the intensity of the first emission light L2A and the intensity of the second emission light L2B. The conversion information D21 includes the calibration curve G12 showing a relationship between the Rayleigh scattering intensity obtained from the first light intensity information D11 and the second light intensity information D12 and the "cell concentration" of the cell suspension 3. A "Rayleigh scattering coefficient" is a parameter indicating a relationship between the Rayleigh scattering intensity and the wavelength, and can be determined from the wavelength dependence of the Rayleigh scattering intensity. There is a proportional relationship in which the Rayleigh scattering intensity increases as the Rayleigh scattering coefficient increases. For that reason, it is possible to quantitatively determine the "cell concentration" by determining either the Rayleigh scattering intensity or the Rayleigh scattering coefficient. In the present embodiment, a case of using the Rayleigh scattering coefficient to determine the "cell concentration" is exemplified. The conversion information D21 may include, in addition to the calibration curve G12, information required for converting the first light intensity information D11 and second light intensity information D12 into the "cell concentration." For example, the conversion information D21 may include the intensity of the irradiation light L1A for determining the first amount of change in light intensity from the first light intensity information D11, or may include the intensity of the irradiation light L1B for determining the second amount of change in light intensity from the second light intensity information D12. The conversion information D21 may include the volume of the container 5 or the volume of the culture medium 7 required for determining the "cell number" from the "cell concentration." As shown in Fig. 13, the calibration curve G12 is a graph plotting the relationship between the Rayleigh scattering coefficient and the "cell concentration."

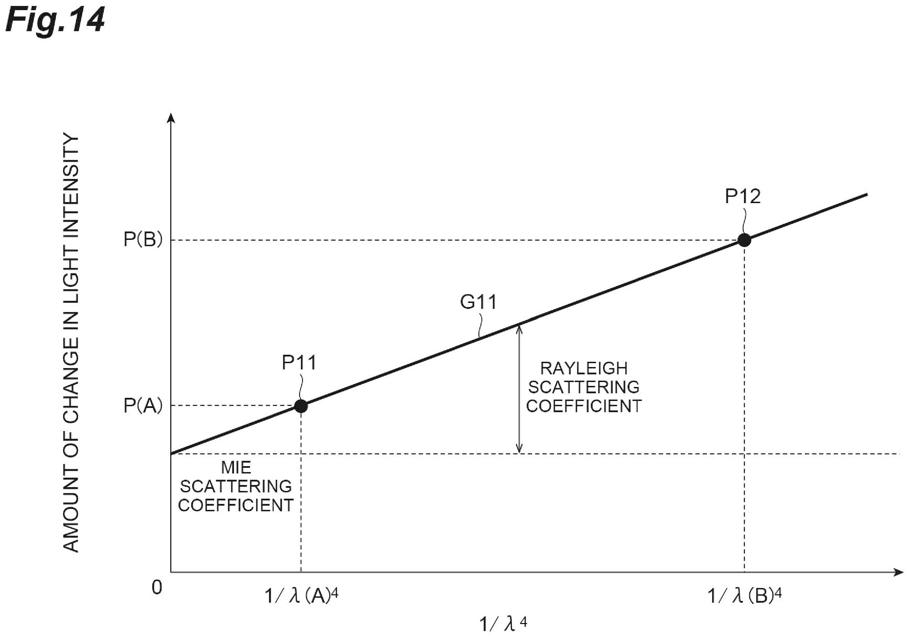

Fig. 14 is a graph for describing how to determine the Rayleigh scattering coefficient. A graph G11 shown in Fig. 14 shows a relationship between the amount of change in light intensity and the wavelength λ of light, when there is no influence of absorption but there are influences of Rayleigh scattering and Mie scattering. In Fig. 14, the vertical axis represents the amount of change in light intensity and the horizontal axis represents the reciprocal of the fourth power of the wavelength λ. Fig. 14 shows a plot P11 showing a relationship between a first amount of change in light intensity P(A) and a first wavelength λ(A), and a plot P12 showing a relationship between a second amount of change in light intensity P(B) and a second wavelength λ(B). The first amount of change in light intensity P(A) is a relative ratio of the intensity of the first emission light L2A to the intensity of the first irradiation light L1A, and is defined as -10Log(intensity of first emission light L2A/intensity of first irradiation light L1A). The second amount of change in light intensity P(B) is a relative ratio of the intensity of the second emission light L2B to the intensity of the second irradiation light L1B, and is defined as -10Log(intensity of the second emission light L2B/intensity of the second irradiation light L1B).

In Fig. 14, the wavelength-dependent Rayleigh scattering intensity is expressed as a product of a slope of a line connecting the plot P11 to the plot P12 (that is, the Rayleigh scattering coefficient) and the reciprocal of the fourth power of the wavelength λ. Accordingly, a Rayleigh scattering coefficient Pr1 can be determined by the following equation (1). Once the Rayleigh scattering coefficient Pr1 is determined, the Rayleigh scattering intensity can also be determined using the above relationship.
[Math. 1] $Pr 1 = \left|\frac{P \left(A\right) - P \left(B\right)}{\frac{1}{λ {\left(A\right)}^{4}} - \frac{1}{λ {\left(B\right)}^{4}}}\right|$ (1)

Fig. 12 will be referred to again. The storage unit 133 stores in advance the conversion information D21 including the calibration curve G12 showing the relationship between the Rayleigh scattering coefficient and the "cell concentration." By preparing beforehand a sample of the cell suspension 3 with a known "cell concentration" (or a sample having a "cell concentration" serving as a reference) and irradiating the sample with the first irradiation light L1A and the second irradiation light L1B using the observation device 101, the calibration curve G12 showing the relationship between the Rayleigh scattering coefficient and the "cell concentration" of the sample can be created in advance.

The conversion unit 135 determines the "cell concentration" of the cell suspension 3 using the first light intensity information D11, the second light intensity information D12, and the conversion information D21. Specifically, the conversion unit 135 calculates the first amount of change in light intensity P(A), which is a relative ratio between the intensity of the first emission light L2A and the intensity of the first irradiation light L1A, and the second amount of change in light intensity P(B), which is a relative ratio between the intensity of the second emission light L2B and the intensity of the second irradiation light L1B. Next, the conversion unit 135 calculates the Rayleigh scattering coefficient Pr1 shown in Equation (1) using the first amount of change in light intensity P(A) and the second amount of change in light intensity P(B). After that, the conversion unit 135 converts the Rayleigh scattering coefficient Pr1 to the "cell concentration" by referring to the calibration curve G12. Thus, the "cell concentration" can be quantitatively determined on the basis of the intensity of the first emission light L2A and the intensity of the first irradiation light L1A.

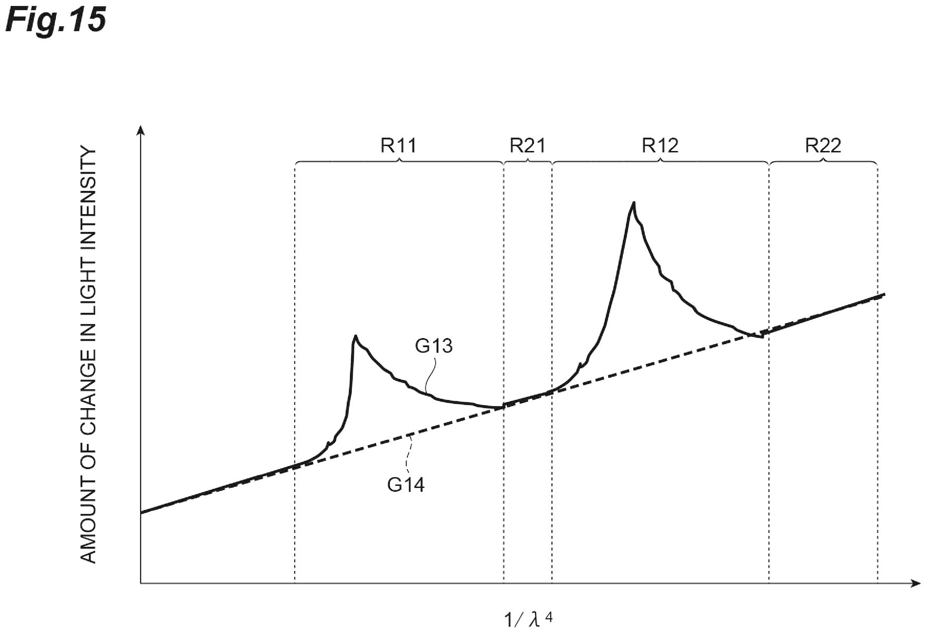

Fig. 15 is a graph for describing a wavelength range suitable for observing the state of the cell suspension 3. A graph G13 represented by the solid line in Fig. 15 shows the wavelength dependence of the amount of change in light intensity, which reflects the influences of Rayleigh scattering and Mie scattering by the cells 2 and light absorption by the cells 2 and the culture medium 7. A graph G14 represented by the dotted line in Fig. 15 shows the wavelength dependence of the amount of change in light intensity when there is no influence of light absorption by the cells 2 and the culture medium 7 (that is, there are only the influences of Rayleigh scattering and Mie scattering by the cells 2). As shown in Fig. 15, the graph G13 includes wavelength ranges R11 and R12 in which peaks with sharply increasing amount of change in light intensity exist, and wavelength ranges R21 and R22 in which no such peaks exist. In the wavelength ranges R11 and R12 in which such peaks exist, it may be difficult to accurately determine the Rayleigh scattering coefficient due to the influence of the peaks. Accordingly, in order to accurately determine the Rayleigh scattering coefficient, it is appropriate to use wavelengths included in the wavelength ranges R21 and R22 in which no peaks exist.

In the present embodiment, the first wavelength λ(A) of the first irradiation light L1A and the second wavelength λ(B) of the second irradiation light L1B are both included in the wavelength range R2 (see Fig. 6) of 650 nm to 850 nm and 1060 nm to 1090 nm. In the graph G13 in Fig. 15, wavelengths of 1060 nm to 1090 nm are included in the wavelength range R21 in which no peaks exist, and wavelengths of 650 nm to 850 nm are included in the wavelength range R22 in which no peaks exist. Accordingly, in the case of irradiating the cell suspension 3 with the first irradiation light L1A and the second irradiation light L1B described above, the amount of change in light intensity without being affected by the absorption of the irradiation light L1 by the cells 2 and the culture medium 7 can be obtained, and thus it is possible to accurately determine the Rayleigh scattering coefficient on the basis of the amount of change in light intensity.

Effects obtained by the second embodiment described above will be described. As described above, light scattering that occurs in particles such as the cells 2 when light is irradiated onto the cell suspension 3 includes Rayleigh scattering caused by particles with sizes smaller than the wavelength of light and Mie scattering by particles with sizes equal to or larger than the wavelength of light. If the cells 2 in the cell suspension 3 adhere to each other to form a cell cluster, the amount and structure of molecules forming the cell membrane of the cells 2 in the cell cluster do not change, and thus the Rayleigh scattering intensity obtained from the cell suspension 3 upon light irradiation does not change. Since the sizes of the particles (cell cluster) formed by the adhesion of the cells 2 to each other change, the Mie scattering intensity obtained from the cell suspension 3 changes. In this case, it is assumed that the intensity of the light emitted from the cell suspension 3 changes even though the number of cells 2 in the cell suspension 3 remains unchanged. Such changes in the intensity of the emission light can cause a decrease in accuracy of estimating the "cell concentration." Resin spheres that can be used as scaffold materials for culturing the cells 2 have a homogeneous molecular structure, and thus when light hits these resin spheres, only Mie scattering occurs. Such scaffold materials can also cause a decrease in accuracy of estimating the "cell concentration."

Accordingly, if only Rayleigh scattering can be observed among scatterings occurring in the cell suspension 3, the "cell concentration" can be accurately determined without being affected by the adhesion between the cells 2 or by the scaffold materials. While the Rayleigh scattering intensity depends on the wavelength of light, the Mie scattering intensity does not depend on the wavelength of light, or does not depend significantly on the wavelength of light. For that reason, in the case of observing the state of the cell suspension 3 using light of a plurality of wavelengths, by utilizing this difference in wavelength dependence, only the wavelength-dependent Rayleigh scattering intensity can be determined, and thus it is possible to accurately determine the "cell concentration."

As in the present embodiment, the irradiation unit 110 may include the first light source 113A, the second light source 113B, and the switching unit 117. The light receiving unit 120 may include the optical sensor 123. In this case, the intensity of the first emission light L2A and the intensity of the second emission light L2B can be detected separately using one optical sensor 123. As a result, there is no need to separately prepare an optical sensor for detecting the intensity of the first emission light L2A and an optical sensor for detecting the intensity of the second emission light L2B, and thus complication of the configuration of the light receiving unit 120 can be avoided.

The observation device and the observation method of the present disclosure are not limited to the second embodiment described above, and various other modifications are possible.

### <Modified Example 1>

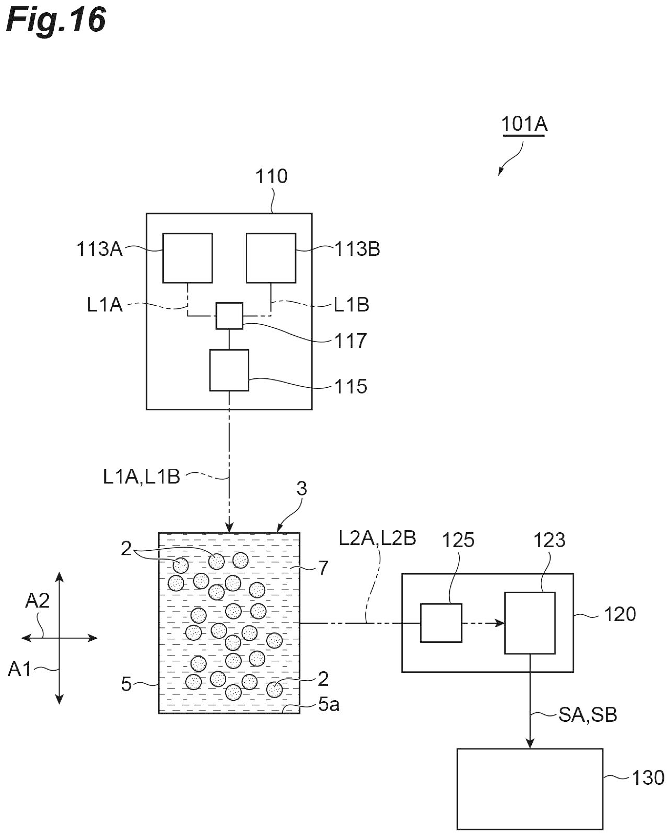

Fig. 16 is a diagram showing a modified example of the observation device 101. As in an observation device 101A shown in Fig. 16, the light receiving unit 120 may be disposed at a position facing the cell suspension 3 in the lateral direction A2. In this case, the light receiving unit 120 sequentially receives the first emission light L2A and the second emission light L2B (hereinafter simply referred to as "emission lights L2A and L2B") emitted from the cell suspension 3 in the lateral direction A2 as a result of radiating the first irradiation light L1A and the second irradiation light L1B (hereinafter simply referred to as "irradiation lights L1A and L1B") from the irradiation unit 110. The emission lights L2A and L2B are scattered light of the irradiation lights L1A and L1B scattered by the cell suspension 3. The light receiving unit 120 is disposed on the optical paths of the emission lights L2A and L2B extending from the cell suspension 3 in the lateral direction A2.

The light receiving unit 120 receives the emission lights L2A and L2B through the lens 125 and outputs the first electric signal SA indicating the intensity of the first emission light L2A and the second electric signal SB indicating the intensity of the second emission light L2B to the analysis unit 130. The optical axes of the emission lights L2A and L2B are, for example, parallel to the lateral direction A2 and perpendicular to the vertical direction A1 along which the irradiation lights L1A and L1B extend. As in the second embodiment, the analysis unit 130 determines the Rayleigh scattering coefficient on the basis of the intensities of the emission lights L2A and L2B.

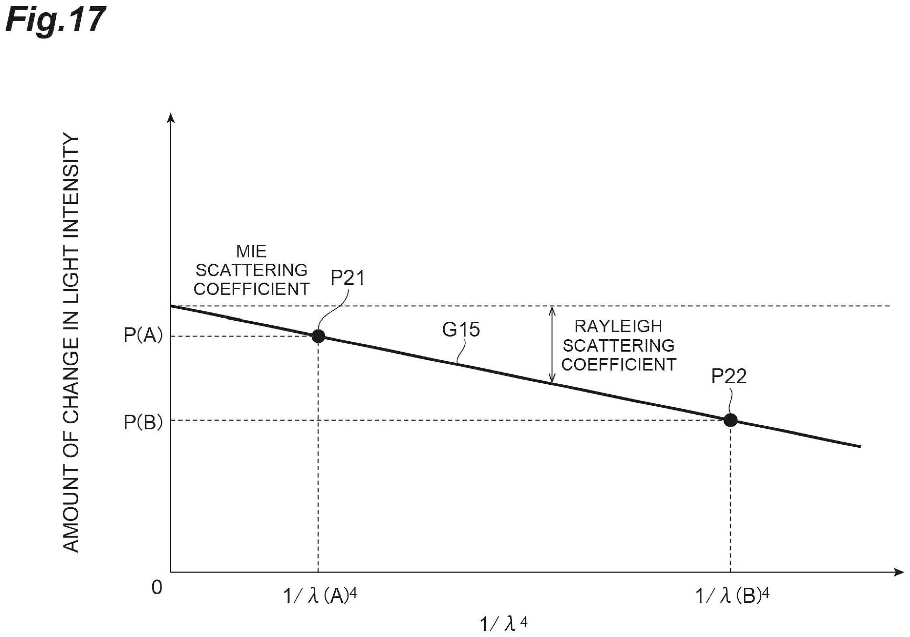

Fig. 17 is a graph for describing how to determine the Rayleigh scattering coefficient. In Fig. 17, the vertical axis represents the amount of change in light intensity, and the horizontal axis represents the reciprocal of the fourth power of the wavelength λ. Fig. 17 shows a plot P21 showing a relationship between the first amount of change in light intensity P(A) and the first wavelength λ(A) of the first emission light L2A, and a plot P22 showing a relationship between the second amount of change in light intensity P(B) and the second wavelength λ(B) of the second emission light L2B. The first amount of change in light intensity P(A) is a relative ratio of the intensity of the first emission light L2A to the intensity of the first irradiation light L1A, and is defined as -10Log(intensity of first emission light L2A/intensity of first irradiation light L1A). The second amount of change in light intensity P(B) is a relative ratio of the intensity of the second emission light L2B to the intensity of the second irradiation light L1B, and is defined as -10Log(intensity of the second emission light L2B/intensity of the second irradiation light L1B).

In a graph G15 shown in Fig. 17, the wavelength-dependent Rayleigh scattering intensity is expressed as a product of a slope of a line connecting the plot P21 to the plot P22 (that is, the Rayleigh scattering coefficient) and the reciprocal of the fourth power of the wavelength λ. Accordingly, the Rayleigh scattering coefficient Pr1 can be determined using the above-mentioned equation (1). After determining the Rayleigh scattering coefficient Pr1, the analysis unit 130 converts the Rayleigh scattering coefficient Pr1 into the "cell concentration" by referring to the calibration curve G12 shown in Fig. 13. Accordingly, since the observation device 101A can also accurately determine the "cell concentration" on the basis of the intensities of the emission lights L2A and L2B having different wavelengths from each other, the same effects as the second embodiment can be obtained.

### <Modified Example 2>

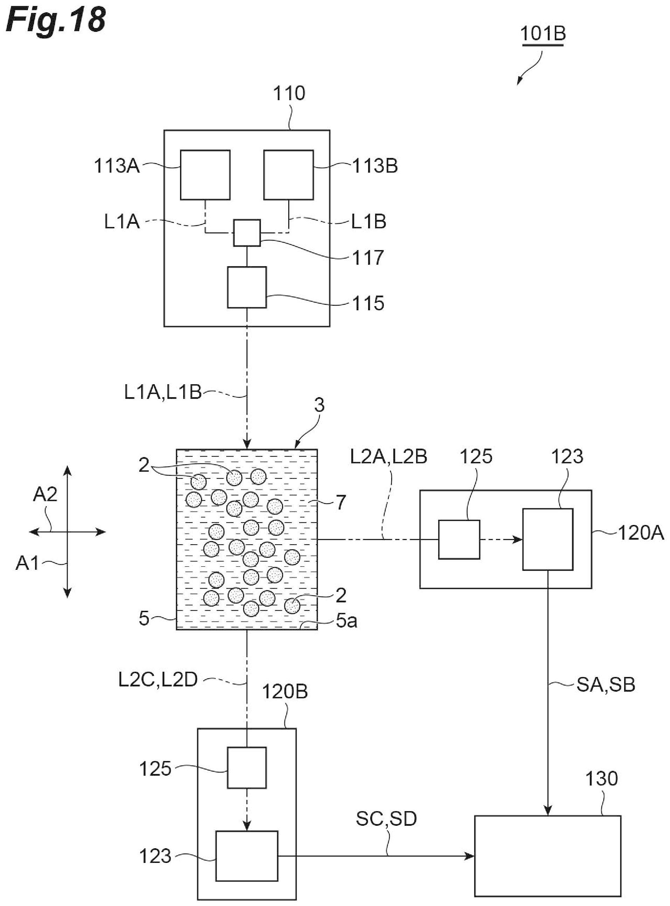

Fig. 18 is a diagram showing a modified example of the observation device 101. As shown in Fig. 18, an observation device 101B may include a first light receiving unit 120A and a second light receiving unit 120B. The first light receiving unit 120A has the same configuration as the light receiving unit 120 shown in Fig. 16. That is, the first light receiving unit 120A is disposed at a position facing the cell suspension 3 in the lateral direction A2 and receives scattered light of the irradiation lights L1A and L1B radiated to the cell suspension 3, which is scattered by the cell suspension 3, as the emission lights L2A and L2B. The first light receiving unit 120A outputs the first electric signal SA indicating the intensity of the first emission light L2A and the second electric signal SB indicating the intensity of the second emission light L2B to the analysis unit 130.

The second light receiving unit 120B has the same configuration as the light receiving unit 120 of the second embodiment described above. That is, the second light receiving unit 120B is disposed at a position facing the irradiation unit 110 in the vertical direction A1 with the cell suspension 3 interposed therebetween, and receives transmitted light of the irradiation lights L1A and L1B radiated to the cell suspension 3, in which is transmitted through the cell suspension 3, as third emission light L2C and fourth emission light L2D (hereinafter simply referred to as "emission lights L2C and L2D"). The second light receiving unit 120B outputs a third electric signal SC indicating an intensity of the third emission light L2C and a fourth electric signal SD indicating an intensity of the fourth emission light L2D to the analysis unit 130.

The analysis unit 130 determines the "cell concentration" of the cell suspension 3 on the basis of the intensities of the emission lights L2A, L2B, L2C, and L2D. For example, after determining the Rayleigh scattering coefficient using the emission lights L2A and L2B, the analysis unit 130 converts the Rayleigh scattering coefficient into the "cell concentration" by referring to the calibration curve G12 shown in Fig. 13. Similarly, after determining the Rayleigh scattering coefficient using the emission lights L2C and L2D, the analysis unit 130 converts the Rayleigh scattering coefficient into the "cell concentration" by referring to the calibration curve G12 shown in Fig. 13. The analysis unit 130 may determine a more accurate "cell concentration" by averaging the two "cell concentrations" determined in this way.

Since the observation device 101B shown in Fig. 18 can also quantitatively determine the "cell concentration" on the basis of the intensities of the emission lights L2A, L2B, L2C, and L2D, the same effects as those of the second embodiment described above can be obtained. In the observation device 101B, by acquiring both the intensities of the emission lights L2A and L2B and the intensities of the emission lights L2C and L2D, the "cell concentration" can be more accurately determined on the basis of these intensities.

### <Modified Example 3>

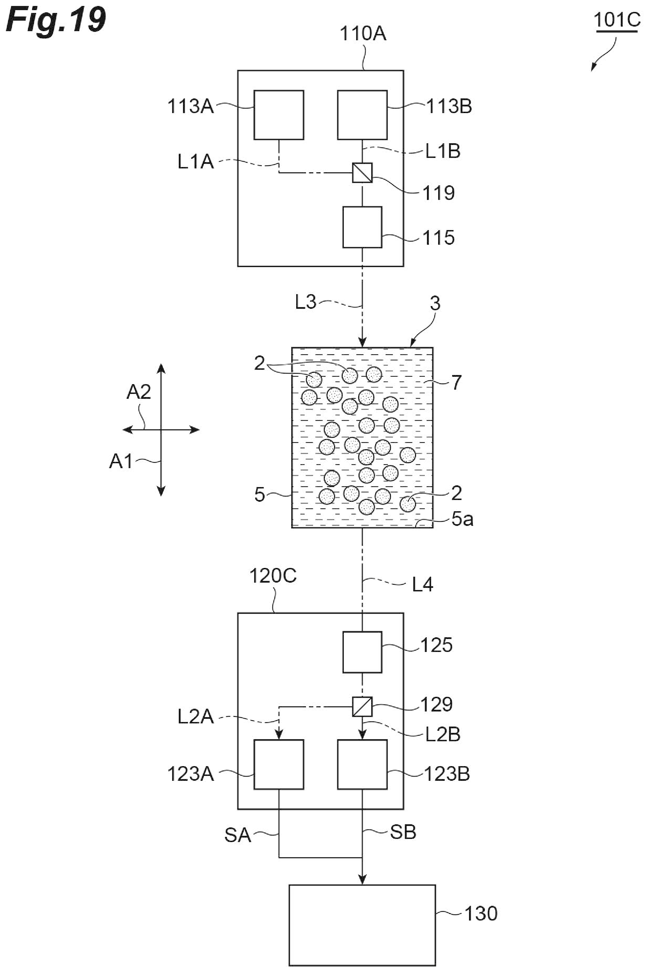

Fig. 19 is a diagram showing a modified example of the observation device 101. As in an observation device 101C shown in Fig. 19, an irradiation unit 110A may include a combining unit 119 instead of the switching unit 117. The combining unit 119 is disposed between the first and second light sources 113A and 113B and the lens 115. The combining unit 119 is optically coupled to the first light source 113A, the second light source 113B, and the lens 115 using lenses and optical fibers. The combining unit 119 combines the first irradiation light L1A from the first light source 113A and the second irradiation light L1B from the second light source 113B to form combined irradiation light L3 and irradiate the cell suspension 3 with the combined irradiation light L3. The combined irradiation light L3 is combined light including the first irradiation light L1A and the second irradiation light L1B, and has the first wavelength λ(A) and the second wavelength λ(B). When the combined irradiation light L3 is radiated onto the cell suspension 3, part of the combined irradiation light L3 is transmitted through the cell suspension 3 and is emitted from the cell suspension 3 as combined emission light L4. The combined emission light L4 has wavelengths corresponding to the first wavelength λ(A) and the second wavelength λ(B) (for example, the same wavelengths as the first wavelength λ(A) and the second wavelength λ(B)). The combining unit 119 is, for example, a dichroic mirror. The combining unit 119 may be an optical fiber type multiplexer, such as a WDM coupler.

The light receiving unit 120C includes a spectroscopic unit 129, a first optical sensor 123A, and a second optical sensor 123B, instead of the optical sensor 123. The first optical sensor 123A and the second optical sensor 123B are disposed below the cell suspension 3 and are adjacent to each other in the lateral direction A2. The spectroscopic unit 129 is disposed between the lens 125 and the first and second optical sensors 123A and 123B. The spectroscopic unit 129 is optically coupled to the lens 125, the first optical sensor 123A, and the second optical sensor 123B using lenses and optical fibers. The spectroscopic unit 129 spectrally separates the combined emission light L4 into respective wavelengths. For example, the spectroscopic unit 129 spectrally separates the combined emission light L4 into the first emission light L2A having the first wavelength λ(A) and the second emission light L2B having the second wavelength λ(B). The spectroscopic unit 129 is, for example, a dichroic mirror. The spectroscopic unit 129 may be an optical fiber type demultiplexer such as a WDM coupler.

The first optical sensor 123A receives the first emission light L2A spectrally separated from the combined emission light L4 by the spectroscopic unit 129 and outputs the first electric signal SA indicating the intensity of the first emission light L2A. The second optical sensor 123B receives the second emission light L2B spectrally separated from the combined emission light L4 by the spectroscopic unit 129 and outputs the second electric signal SB indicating the intensity of the second emission light L2B.

Since the observation device 101C shown in Fig. 19 can also quantitatively determine the "cell concentration" of the cell suspension 3 on the basis of the intensity of the first emission light L2A and the intensity of the second emission light L2B, the same effects as the second embodiment can be obtained. According to the observation device 101C, it is possible to detect the intensity of the first emission light L2A and the intensity of the second emission light L2B simultaneously using the combined irradiation light L3 and the combined emission light L4, and thus the state of the cell suspension 3 based on the intensity of the first emission light L2A and the intensity of the second emission light L2B can be observed more efficiently in a shorter amount of time.

### <Modified Example 4>

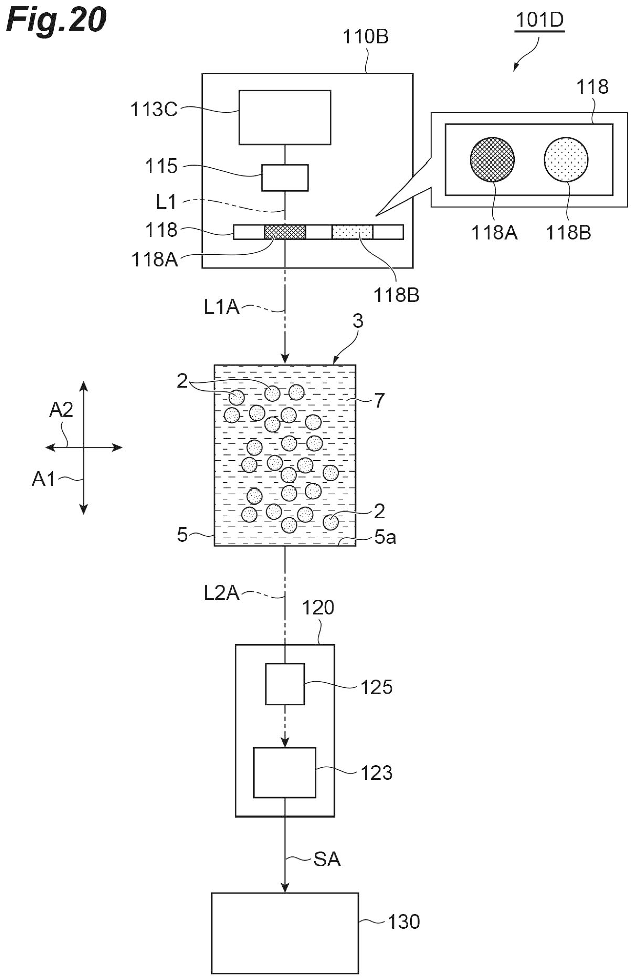

Fig. 20 is a diagram showing a modified example of the observation device 101. As shown in Fig. 20, an observation device 101D may include an irradiation unit 110B instead of the irradiation unit 110. The irradiation unit 110B includes a light source 113C, the lens 115, and a filter switching unit 118. The light source 113C is a halogen lamp (lamp light source) that emits the irradiation light L1 including a plurality of wavelengths. At least two of the wavelengths included in the irradiation light L1 are included in the ranges of 650 nm to 850 nm and 1060 nm to 1090 nm. Other wavelengths included in the irradiation light L1 may be included in the ranges of 650 nm to 850 nm and 1060 nm to 1090 nm, or may be outside the ranges of 650 nm to 850 nm and 1060 nm to 1090 nm.

The lens 115 is optically coupled to the light source 113C using an optical fiber such as a bundle fiber. The filter switching unit 118 is disposed on the optical path of the irradiation light L1 between the lens 115 and the cell suspension 3. The filter switching unit 118 holds a first filter 118A and a second filter 118B, and disposes either the first filter 118A or the second filter 118B on the optical path of the irradiation light L1. The first filter 118A is a bandpass filter that transmits only the first wavelength λ(A) of the plurality of wavelengths included in the irradiation light L1. The second filter 118B is a bandpass filter that transmits only the second wavelength λ(B) of the plurality of wavelengths included in the irradiation light L1. As shown in the upper right corner of Fig. 20, when the filter switching unit 118 is viewed in the vertical direction A1, each of the first filter 118A and the second filter 118B has a circular shape, for example.

As shown in Fig. 20, when the filter switching unit 118 has the first filter 118A disposed on the optical path of the irradiation light L1, the first irradiation light L1A having the first wavelength λ(A) transmitted through the first filter 118A is radiated to the cell suspension 3. When the filter switching unit 118 has the second filter 118B disposed on the optical path of the irradiation light L1, the second irradiation light L1B having the second wavelength λ(B) transmitted through the second filter 118B is radiated to the cell suspension 3. The filter switching unit 118 switches the arrangement of the first filter 118A and the second filter 118B so that the first irradiation light L1A and the second irradiation light L1B are selectively radiated to the cell suspension 3. As described above, the first wavelength λ(A) is included in the ranges of 650 nm to 850 nm and 1060 nm to 1090 nm. The second wavelength λ(B) is a wavelength different from the first wavelength (A) in the ranges of 650 nm to 850 nm and 1060 nm to 1090 nm.

When the first irradiation light L1A is radiated onto the cell suspension 3, part of the first irradiation light L1A is transmitted through the cell suspension 3 and is emitted from the cell suspension 3 as the first emission light L2A. When the second irradiation light L1B is radiated onto the cell suspension 3, part of the second irradiation light L1B is transmitted through the cell suspension 3 and is emitted from the cell suspension 3 as the second emission light L2B. The light receiving unit 120 selectively receives the first emission light L2A and the second emission light L2B emitted from the cell suspension 3. The light receiving unit 120 outputs the first electric signal SA corresponding to the intensity of the first emission light L2A and the second electric signal SB corresponding to the intensity of the second emission light L2B to the analysis unit 130.

Since the observation device 101D shown in Fig. 20 can also quantitatively determine the "cell concentration" of the cell suspension 3 on the basis of the intensity of the first emission light L2A and the intensity of the second emission light L2B, the same effects as the second embodiment can be obtained. When a broadband halogen lamp is used, as in the observation device 101D, the device can be configured more inexpensively than when a laser light source is used.

The present disclosure is not limited to the above-described embodiments and modified examples, and various other modifications are possible. For example, each of the embodiments and each of the modified examples described above may be combined with each other to the extent that they are compatible, depending on the required purpose and effect. The observation device of the present disclosure is not limited to the configurations of each of the embodiments and modified examples described above.

For example, the observation device may include a plurality of irradiation units and a plurality of light receiving units. In this case, the plurality of irradiation units may be arranged two-dimensionally when viewed from above, and may each irradiate each of the plurality of irradiation regions set in the cell suspension with the irradiation light. The plurality of irradiation regions may be a plurality of regions set by dividing the cell suspension when the cell suspension is viewed from above, and may be regions serving as irradiation targets irradiated with the irradiation light. The plurality of irradiation units may be respectively disposed above the plurality of irradiation regions, or may be respectively opposite to the plurality of irradiation regions in a one-to-one relationship in the vertical direction. The plurality of light receiving units may be arranged in a two-dimensional arrangement when viewed from above. The plurality of light receiving units may be disposed below the plurality of irradiation regions, or may be respectively opposite to the plurality of irradiation units in a one-to-one relationship in the vertical direction with the cell suspension interposed therebetween.

In each of the above embodiments and each of the above modified examples, a case in which the irradiation light radiated to the cell suspension includes at least one wavelength in the ranges of 650 nm to 850 nm and 1060 nm to 1090 nm has been described. However, all wavelengths of the irradiation light may be included in the ranges of 650 nm to 850 nm and 1060 nm to 1090 nm. That is, the irradiation light may have only wavelengths in the ranges of 650 nm to 850 nm and 1060 nm to 1090 nm. In this case, a decrease in measurement accuracy due to the presence of wavelengths outside the ranges of 650 nm to 850 nm and 1060 nm to 1090 nm mixed in the wavelengths of the irradiation light can be avoided. The analysis unit may only handle information (irradiation light intensity or emission light intensity) obtained using wavelengths within the ranges of 650 nm to 850 nm and 1060 nm to 1090 nm.

### Reference Signs List

1, 1A, 1B, 101, 101A, 101B, 101C, 101D Observation device
2 Cell
3 Cell suspension
5 Container
5a Bottom surface
7 Culture medium
10, 110, 110A, 110B Irradiation unit
13 Light source (laser light source)
15, 25, 115, 125 Lens
20, 120 Light receiving unit
20A, 120A First light receiving unit
20B, 120B Second light receiving unit
23, 123 Optical sensor
30, 130 Analysis unit
31, 131 Acquisition unit
33, 133 Storage unit
35, 135 Conversion unit
113A First light source
113B Second light source
113C Light source
117 Switching unit
118 Filter switching unit
118A First filter
118B Second filter
119 Combining unit
123A First optical sensor
123B Second optical sensor
129 Spectroscopic unit
301 Processor
302 Main storage device
303 Auxiliary storage device
304 Input device
305 Output device
A1 Vertical direction
A2 Lateral direction
D1 Light intensity information
D2, D21 Conversion information
D11 First light intensity information
D12 Second light intensity information
G1, G10, G12 Calibration curve
L1 Irradiation light
L1A First irradiation light
L1B Second irradiation light
L2 Emission light
L2A First emission light
L2B Second emission light
L2C Third emission light
L2D Fourth emission light
L3 Combined irradiation light
L4 Combined emission light
P(A) First amount of change in light intensity
P(B) Second amount of change in light intensity
Pr1 Rayleigh scattering coefficient
R1, R2, R11, R21, R22 Wavelength range
λ Wavelength
λ(A) First wavelength
λ(B) Second wavelength
S Electric signal
SA First electric signal
SB Second electric signal

## Claims

1. An observation device configured to observe a cell suspension including a plurality of cells, comprising:
at least one irradiation unit that is disposed at a position facing the cell suspension and irradiates the cell suspension with irradiation light having at least one wavelength included in the ranges of 650 nm to 850 nm and 1060 nm to 1090 nm;
at least one light receiving unit that is disposed at a position facing the cell suspension and receives emission light emitted from the cell suspension as a result of irradiating the cell suspension with the irradiation light; and
an analysis unit that is communicatively connected to the light receiving unit and determines a parameter relating to a cell concentration of the cells in the cell suspension on the basis of an intensity of the emission light.

2. The observation device according to claim 1, wherein the irradiation unit includes a light source using a semiconductor light emitting element that emits light having one wavelength included in the ranges of 650 nm to 850 nm and 1060 nm to 1090 nm as the irradiation light.

3. The observation device according to claim 1 or 2, wherein the light receiving unit is disposed at a position opposite to the irradiation unit with the cell suspension interposed therebetween, and receives part of the irradiation light transmitted through the cell suspension as the emission light.

4. The observation device according to any one of claims 1 to 3, wherein
the irradiation unit irradiates the cell suspension with first irradiation light having a first wavelength included in the ranges of 650 nm to 850 nm and 1060 nm to 1090 nm, and second irradiation light having a second wavelength that is included in the ranges of 650 nm to 850 nm and 1060 nm to 1090 nm and is different from the first wavelength,
the light receiving unit receives first emission light and second emission light, which have different wavelengths from each other, and which are emitted from the cell suspension as a result of radiating the first irradiation light and the second irradiation light, and
the analysis unit determines the parameter relating to the cell concentration on the basis of an intensity of the first emission light and an intensity of the second emission light.

5. The observation device according to claim 4, wherein
the irradiation unit includes
a first light source configured to emit the first irradiation light,
a second light source configured to emit the second irradiation light, and
a switching unit configured to switch between an optical path of the first irradiation light and an optical path of the second irradiation light so that the first irradiation light and the second irradiation light are selectively radiated to the cell suspension, and
the light receiving unit includes
an optical sensor configured to selectively receive the first emission light and the second emission light emitted from the cell suspension.

6. The observation device according to claim 4, wherein
the irradiation unit includes
a first light source configured to emit the first irradiation light,
a second light source configured to emit the second irradiation light, and
a combining unit configured to combine the first irradiation light and the second irradiation light to generate combined irradiation light and irradiate the cell suspension with the combined irradiation light, and
the light receiving unit includes
a spectroscopic unit configured to spectrally separate the combined emission light emitted from the cell suspension as a result of radiating the combined irradiation light into respective wavelengths,
a first optical sensor configured to detect the intensity of the first emission light spectrally separated from the combined emission light, and
a second optical sensor configured to detect the intensity of the second emission light spectrally separated from the combined emission light.

7. The observation device according to claim 4, wherein
the irradiation unit includes
a light source configured to emit light including the first irradiation light and the second irradiation light, and
a filter switching unit that includes a first filter configured to transmit only the first irradiation light and a second filter configured to transmit only the second irradiation light, and switches the positions of the first filter and the second filter so that the first filter or the second filter is disposed on an optical path of the light between the light source and the cell suspension. and
the light receiving unit includes
an optical sensor configured to selectively receive the first emission light and the second emission light emitted from the cell suspension.

8. An observation method configured to observe a cell suspension including a plurality of cells, comprising the steps of:
irradiating the cell suspension with irradiation light having at least one wavelength included in the ranges of 650 nm to 850 nm and 1060 nm to 1090 nm;
receiving emission light emitted from the cell suspension as a result of irradiating the cell suspension with the irradiation light; and
determining a parameter relating to a cell concentration of the cells in the cell suspension on the basis of an intensity of the emission light.

9. The observation method according to claim 8, wherein
in the step of irradiating the cell suspension,
first irradiation light having a first wavelength included in the ranges of 650 nm to 850 nm and 1060 nm to 1090 nm, and second irradiation light having a second wavelength included in the ranges of 650 nm to 850 nm and 1060 nm to 1090 nm and different from the first wavelength are radiated to the cell suspension,
in the step of receiving the emission light,
first emission light and second emission light, which have different wavelengths from each other, and which are emitted from the cell suspension as a result of radiating the first irradiation light and the second irradiation light, are received, and
in the step of determining the parameter relating to the cell concentration,
the parameter relating to the cell concentration is determined on the basis of an intensity of the first emission light and an intensity of the second emission light.
